# EUROPEAN PATENT SPECIFICATION

(11) **EP 3 542 810 B1**
(45) Date of publication and mention of the grant of the patent: **24.05.2023**
(21) Application number: 17871096.8
(22) Date of filing: 15.11.2017
(51) Int. Cl.: A61K 36/282, A61K 31/365, A61K 31/198, A61K 31/201, A23L 33/105, A61K 8/97, A61Q 19/00, A61P 17/00, A61P 17/04

(54) **COMPOSITION CONTAINING ARTEMISIA ANNUA EXTRACT AS EFFECTIVE INGREDIENT FOR ALLEVIATING SKIN DISEASE AND PREPARATION METHOD THEREFOR**
ZUSAMMENSETZUNG MIT EXTRAKT AUS ARTEMISIA ANNUA ALS WIRKSTOFF ZUR LINDERUNG VON HAUTKRANKHEITEN UND HERSTELLUNGSVERFAHREN DAFÜR
COMPOSITION CONTENANT UN EXTRAIT D'ARMOISE ANNUELLE COMME AGENT DERMATOLOGIQUE ET SA PREPARATION

(30) Priority: 15.11.2016 KR 20160152229; 13.07.2017 WO PCT/KR2017/007500
(43) Date of publication of application: 25.09.2019
(73) Proprietor: Korea Institute of Science and Technology, Seoul 02792 (KR); Gachon University of Industry-Academic Cooperation Foundation, Seongnam-si, Gyeonggi-do 13120 (KR)
(72) Inventor: KWON, Hak Cheol, Gangneung-si Gangwon-do 25451 (KR); HAM, Jungyeob, Gangneung-si Gangwon-do 25451 (KR); LEE, Jae Wook, Seoul 02792 (KR); PARK, Jin Soo, Gangneung-si Gangwon-do 25451 (KR); LEE, Ju Young, Seoul 02792 (KR); PARK, Young Tae, Gangneung-si Gangwon-do 25451 (KR); CHA, Jin Wook, Gangneung-si Gangwon-do 25451 (KR); KWON, Jaeyoung, Gangneung-si Gangwon-do 25451 (KR); KIM, Sung Hun, Gangneung-si Gangwon-do 25451 (KR); PARK, Woong Bi, Gangneung-si Gangwon-do 25451 (KR); HWANG, Ho Seong, Gangneung-si Gangwon-do 25451 (KR); PARK, Jae Gyu, Daegu 42073 (KR); KIM, Sun Yeou, Seoul 06280 (KR); KANG, Min Cheol, Incheon 21932 (KR)
(74) Representative: Gill, Siân Victoria
(86) International application number: PCT/KR2017/012958
(87) International publication number: WO 2018/093150

(56) References cited:
- EP-A1- 0 908 460
- EP-B1- 0 908 460
- WO-A1-2010/012686
- WO-A2-2010/012687
- CN-A- 102 816 065
- JP-A- H1 179 935
- JP-A- H1 179 937
- KR-A- 20020 035 322
- KR-A- 20090 022 902
- KR-A- 20090 081 956
- KR-A- 20120 058 709
- KR-A- 20150 066 098
- US-B1- 6 337 095
- Sun Seo et al.: "Antioxidant Activities of Extracts from Artemisia capillaris THUNB. and Artemisia iwayomogi KITAM. used as Injin", Korean J. Plant Res, 1 January 2008 (2008-01-01), pages 292-298, XP055678978, Retrieved from the Internet: URL:https://pdfs.semanticscholar.org/a01c/ 66abb045b5324e88481a1589568e1441ea50.pdf?_ ga=2.31437049.753489819.1585035148-1971391 602.1554817577 [retrieved on 2020-03-24] -& Sun Seo et al.: "GOOGLE TRANSLATION: Antioxidant Activities of Extracts from Artemisia capillaris T HUNB . and Artemisia iwayomogi K ITAM . used as Injin", Korean J. Plant Res, 1 January 2008 (2008-01-01), pages 292-298, XP055678984, Retrieved from the Internet: URL:https://pdfs.semanticscholar.org/a01c/ 66abb045b5324e88481a1589568e1441ea50.pdf?_ ga=2.224285917.753489819.1585035148-197139 1602.1554817577 [retrieved on 2020-03-24]
- BILIA A R ET AL: "Simultaneous analysis of artemisinin and flavonoids of several extracts of Artemisia annua L. obtained from a commercial sample and a selected cultivar", PHYTOMEDICINE, ELSEVIER, AMSTERDAM, NL, vol. 13, no. 7, 10 July 2006 (2006-07-10), pages 487-493, XP028022210, ISSN: 0944-7113, DOI: 10.1016/J.PHYMED.2006.01.008 [retrieved on 2006-07-10]
- SEO, KYOUNG SUN ET AL.: 'Antioxidant Activities of Extracts from Artemisia capillaris and Artemisia iwayomogi Used as Injin' THE PLANT RESOURCES SOCIETY OF KOREA vol. 21, no. 4, 30 August 2008, pages 292 - 298, XP053017447
- KIM, R.-J. ET AL.: 'Antioxidant and Cancer Cell Growth Inhibition Activity of Five Different Varieties of Artemisia Cultivars in Korea' JOURNAL OF LIFE SCIENCE vol. 22, no. 6, 2012, pages 844 - 851, XP053031121

## Description

### TECHNICAL FIELD

The present invention relates to a composition for use in the prevention or treatment of an inflammatory skin disorder including an *Artemisia annua* extract as an active ingredient, and a method of preparing the extract.

### BACKGROUND ART

Research and development have been carried out on *Artemisia annua* extract. However, there has never been any instance of development of extracts from which low-polar fractions or extracts are removed for use in ameliorating skin itching and dermatitis.

Direct skin contact with antigens may lead to an allergic reaction or skin irritation, which could act as consistent stress factors, destroying homeostasis of the skin barrier and consequently causing itching and inflammation. In particular, atopic dermatitis does not yet have a reliable therapy since its causes are unidentified, and in general, steroidal anti-inflammatory drugs are used to merely temporarily relieve symptoms or moisturizing agents such as moisturizing creams are used to relieve itching. Recently, about 10 % of infants and children are suffering from atopic dermatitis, and the number of patients continues to rise. With the aging of the population, aged dry skin is increasing, leading to an increasing variety of types of dermatitis. Since steroid agents cause side effects and resistance thereto, there is an urgent need for the development of non-steroidal natural drugs for the treatment of dermatitis, including atopic dermatitis.

According to the related art, research has been carried out on *Artemisia* extracts including artemisinin or sesquiterpene lactone, or essential oil ingredients, but a dermatitis amelioration effect of *Artemisia* extracts from which most sesquiterpene lactone including artemisinin and essential oil ingredients are removed, as in the present invention, is not yet known.

WO 2010/012686 discloses a method for producing surfactant-free emulsions from the annual mugwort (*Artemisia annua*) and fungi of the genus ganoderma, which are characterised by improved properties, especially free-radical scavenger properties and an anti-aging effect, and can therefore have multiple uses in cosmetics.

WO 2010/012687 discloses preparations based on *Artemisia annua,* and to a plurality of methods for producing microparticles and nanoparticles from the annual mugwort (*Artemisia annua*). Ultrafine particles with improved properties are obtained as a result, that can be used as food supplements and feed supplements, in cosmetics and for medical purposes.

EP 0 908 460 A1 discloses a novel process for the simultaneous production of essential oil and artemisinic acid optionally converted into artemisinin, from the plant *Artemisia annua.* Also disclosed are both the use of artemisinin obtained from said process as an anti-malarial drug, as well as the use of the essential oil produced from the process in perfumery, cosmetics, and aromatherapy.

Bilia A. R., et al, "Simultaneous analysis of artemisinin and flavonoids of several extracts of Artemisia annua L. obtained from a commercial sample and a selected cultivar", Phytomedicine, Elsevier, vol. 13, no. 7, discloses investigations into the effect of several flavonoids of *A. annua* on the reported antimalarial activity of artemisinin.

CN 102816065 discloses the use of *Artemisia annua* and *Artemisia annua* industrial-extraction residues as raw materials for caffeoylquinic acid preparation.

US 6,337,095 discloses a process for the isolation of the compound scopoletin which is used as nitric oxide synthesis inhibitor from *Artemisia annua* and other plant families.

### DETAILED DESCRIPTION OF THE INVENTION

### TECHNICAL PROBLEM

Accordingly, the present inventors provide a composition for use in the prevention or treatment of various inflammatory skin disorders including, as an active ingredient, an *Artemisia annua* extract prepared by the method as defined in claim 1, and the method of isolating the Artemisia annua extract as defined in claim 1.

### TECHNICAL SOLUTION

According to an aspect of the present invention, there is provided a method of isolating an *Artemisia annua* extract, the method including: contacting *Artemisia annua* plant with a low-polarity solvent to extract a component of the *Artemisia annua* into the low-polarity solvent, wherein the low polarity solvent is dichloromethane, diethyl ether, ethyl acetate or acetone; removing the low-polarity solvent from a mixture obtained after the contacting to obtain a residue; and contacting the residue with a hydrophilic solvent to extract a component of the *Artemisia annua* into the hydrophilic solvent, wherein the hydrophilic solvent is a C1-C6 alcohol, water, or a combination thereof, and wherein the *Artemisia annua* extract comprises at least four compounds selected from the group consisting of ornithine, pinitol, muco-inositol, chiro-inositol, betaine, and chlorogenic acid.

According to another aspect of the present invention, there is provided a composition for use in the prevention or treatment of a skin disorder, the composition including the *Artemisia annua* extract prepared by the above-described method, wherein the skin disorder is at least one inflammatory skin disorder selected from the group consisting of skin pruritus, seborrheic dermatitis, contact dermatitis, deciduous dermatitis, hidradenitis, atopic dermatitis, drug allergy, diabetic dermatitis, bacterial dermatitis, fungal dermatitis, allergic dermatitis and neurogenic dermatitis, and wherein the *Artemisia annua* extract comprises at least four compounds selected from the group consisting of ornithine, pinitol, muco-inositol, chiro-inositol, betaine, and chlorogenic acid.

According to a preferred aspect of the present invention, the composition for use is in the form of a pharmaceutical composition, a quasi-drug composition, a food composition or a cosmetic composition which includes an *Artemisia annua* extract prepared by the above-described *Artemisia annua* extract isolation method.

### DESCRIPTION OF THE DRAWINGS

FIG. 1 illustrates chromatograms of high-performance liquid chromatography (HPLC) of an *Artemisia annua* ethanol extract (AAMM) obtained from residue remaining after extraction of *Artemisia annua* with dichloromethane.
FIGS. 2 and 3 illustrate HPLC chromatograms of a highly polar fraction, AAMM-fraction 1, prepared in Example 2.
FIGS. 4 and 5 illustrate HPLC chromatograms of AAMM-fraction 4.
FIG. 6 illustrates ¹⁸F-fluorodeoxyglucose (¹⁸F-FDG used as a tracer) positron emission tomography-computed tomography (PET-CT) images of mice of which inflammatory sites were treated with an *Artemisia annua* dichloromethane extract (AAM) and an *Artemisia annua* ethanol extract (AAMM) obtained from the residue remaining after dichloromethane extraction.
FIG. 7 illustrates microscope images showing changes in tissues after inflammatory sites of the mice were treated with AAM and AAMM.
FIG. 8 illustrates histopathological microscope images after hematoxylin and eosin (H&E) staining showing changes in tissues after the treatment of inflammatory sites in mice with 1-fluro-2,4-dinitrobenzene (DNFB), dexamethasone, AAMM, AAMM-fraction 1, AAMM-fraction 2, AAMM-fraction 3, and AAMM-fraction 4 (x200 magnification).
FIG. 9 illustrates microscope images of Picrosirius red (PSR)-stained tissue sections showing collagen distribution changes in tissues after the treatment of inflammatory sites in mice with DNFB, dexamethasone, AAMM, AAMM-fraction 1, AAMM-fraction 2, AAMM-fraction 3, and AAMM-fraction 4 (x100 magnification).
FIG. 10 illustrates microscope images after H&E staining and picrosirius red (PSR) staining, showing changes in tissues after the treatment of inflammatory sites in mice with an *Artemisia annua* ethanol extract (AA) including sesquiterpene lactone and AAMM-1+4, in terms of epidermal thickness, the presence or absence of inflammatory cells, the epidermis, acanthosis, and collagen distribution (x200 magnification).
FIG. 11 illustrates (A) graphs of autophagy induction cell protective activity of a single compound represented by one of Formulae 1 to 7; and (B) graphs of autophagy induction cell protective activity of compositions each including a selective combination of the compounds represented by Formulae 1 to 7, wherein "RPM" denotes rapamycin (as a positive control group), and "C" denotes a negative control group treated with DMSO at a same concentration as applied to drug treatment groups.
FIG. 12 illustrate results of microscopic observation after hematoxylin and eosin (H&E) staining of skin tissue sections in which DNFB-induced inflammatory sites were treated with an AA-M6 composition and an AA-M9 composition.
FIG. 13 illustrates HPLC results of a methanol extract of residue remaining after an extract of *Artemisia annua* originating from China obtained with methylene chloride was removed.
FIG. 14 illustrates HPLC results of an ethanol extract of residue remaining after an extract of *Artemisia annua* originating from China obtained with diethyl ether was removed.
FIG. 15 illustrates HPLC results of an ethanol extract of residue remaining after an extract of *Artemisia annua* originating from China obtained with ethyl acetate was removed.
FIG. 16 illustrates HPLC results of an ethanol extract of residue remaining after an extract of *Artemisia annua* originating from China obtained with acetone was removed.
FIG. 17 illustrates HPLC results of an ethanol extract of residue remaining after an extract of *Artemisia annua* originating from China obtained by supercritical extraction with liquid carbon dioxide was removed.
FIG. 18 illustrates HPLC results of a methanol extract of residue remaining after an extract *Artemisia annua* originating from Korea obtained with methylene chloride was removed.
FIG. 19 illustrates HPLC results of an ethanol extract of residue remaining after an extract of *Artemisia annua* originating from Korea obtained with diethyl ether was removed.
FIG. 20 illustrates HPLC results of an ethanol extract of residue remaining after an extract of *Artemisia annua* originating from Korea obtained with ethyl acetate was removed.
FIG. 21 illustrates HPLC results of an ethanol extract of residue remaining after an extract of *Artemisia annua* originating from Korea obtained with acetone was removed.
FIG. 22 is a graph illustrating the effect of an ethyl acetate extract (AAE1) of *Artemisia annua* and an ethanol extraction (AAEM1) prepared from residue on expression of interleukin-1 β (IL-1 β) in cells.
FIG. 23 illustrates the effect of AAE1 and AAEM1 on expression of LC3 in cells.

### BEST MODE

One or more embodiments of the present invention will now be described in detail with reference to the following examples. However, these examples are only for illustrative purposes and are not intended to limit the scope of the one or more embodiments of the present invention. For the avoidance of doubt, methods comprising contacting *Artemisia annua* plant with liquid carbon dioxide are not claimed.

### Example 1: Preparation of Artemisia annua extract

### 1. Preparation of Artemisia annua extract containing sesquiterpene and essential oil component

A dried aerial part of *Artemisia annua* (grown in China) was purchased, and 5 kg of the sample was finely cut and put into a 50-L glass bottle chosen as an extraction container, 40 L of dichloromethane was added thereto and subject to extraction with shaking at room temperature for about 48 hours. The obtained mixture was filtered by gravity with a Watman filter paper having a thickness of about 0.34 mm and a glass funnel having a diameter of about 30 cm. These extraction and filtering processes were repeated twice to thereby obtain a filtered extract. The filtered extract was put in a vacuum evaporator and then concentrated under reduced pressure at about 30 °C until the solvent was completely evaporated. As a result, about 114 g of *Artemisia annua* dichloromethane extract (hereinafter, referred to also as 'AAM') containing sesquiterpene lactone including artemisinin, and essential oils as major components was obtained (Yield: 2.3 %).

### 2. Preparation of Artemisia annua alcohol extract from which most sesquiterpene and essential oil components are removed

About 40 L of ethanol was added to the residue remaining after the filtration performed on the mixture of dichloromethane and the aerial part in Section 1 above, left at about 45 °C with shaking for about 48 hours, and cooled down to room temperature. The obtained mixture was filtered by gravity with a Watman filter paper having a thickness of about 0.34 mm and a glass funnel having a diameter of about 30 cm. These warm-immersion and filtering processes were repeated twice to thereby obtain a filtered extract. The filtered extract was put in a vacuum evaporator and then concentrated under reduced pressure at about 40 °C until the solvent was completely evaporated. As a result, about 484 g of *Artemisia annua* ethanol extract (hereinafter, referred to also as 'AAMM') from which most the sesquiterpene lactone including artemisinin and essential oil components were removed (Yield: 9.7 %).

The AAMM extract was analyzed by high-performance liquid chromatography (HLPC) under the following analysis conditions. As a result, lower-polar components such as sesquiterpene lactone, sesquiterpene, and essential oils which are observed mainly after the residence time of 14 minutes were found to have very small peaks or nearly zero peaks. Specifically, the AAMM extract contained about 0.001 (w/w)% of sesquiterpene lactone and about 0.01 (w/w)% or less of volatile essential oil components, with respect to a total weight of the AAMM extract. Sesquiterpene lactone includes artemisinin, artemisinic acid, arteannuin, and artemicitin.

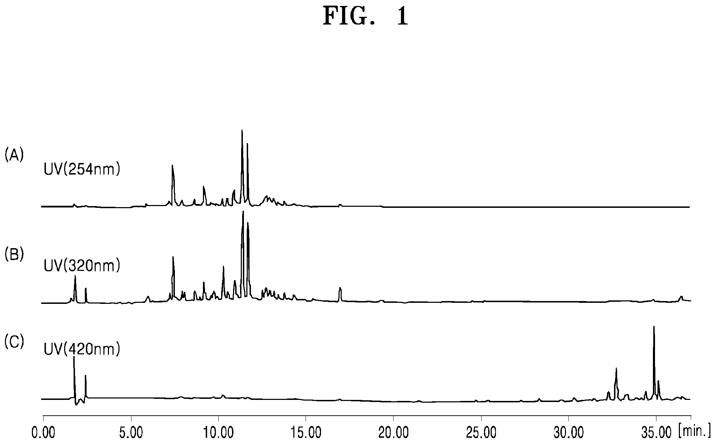

FIG. 1 shows HPLC chromatograms of the *Artemisia annua* ethanol extract extracted from the residue remaining after extraction of *Artemisia annua* with dichloromethane. In FIG. 1, (A), (B) and (C) represents chromatogram obtained with an ultraviolet (UV) detector at a wavelength of 254 nm, 320 nm and 420 nm, respectively. In (C) of FIG. 1, low-polar component peaks mainly detected after 35 minutes were found to be carotenoid and chlorophyll components, by comparison with a reference UV spectrum.

HPLC analysis conditions of the AAMM were as follows.
Equipment used: Waters 1525 HPLC system
Column: RP C-18 HPLC column (4.6x150 mm, 5 µm)
Solvents:
   (a) Acetonitrile containing 0.02% trifluoroacetic acid (TFA)
   (b) Water containing 0.02 % TFA
Mobile phase composition: Elution starts with a 10:90 ratio by volume of solvent (a) to solvent (b) at 0 minutes, then with an increasing ratio of volume of solvent (a) from 10 % to 100 % at 0 minutes to 30 minutes, and then with 100 % by volume of solvent (a) at 30 minutes to 37 minutes
Mobile phase flow rate: 1.0 mL/min
Detection wavelength of detector: UV - 254 nm, 320 nm, and 420 nm

### 3. Preparation of Artemisia annua ethanol extract

Dried whole *Artemisia annua* (grown in China) was purchased, and 200 g of the sample was finely cut and put into an extraction container. About 1.6 L of ethanol was added thereto, left about 45 °C with shaking for about 48 hours, cooled down to room temperature, and then filtered. These processes were repeated twice to thereby obtain a filtered extract. The filtered extract was put in a vacuum evaporator and then concentrated under reduced pressure at about 40 °C until the solvent was completely evaporated. As a result, about 23 g of *Artemisia* annua ethanol extract (hereinafter, also referred to as 'AA') including artemisinin, sesquiterpene lactone, and essential oil components, and most the components of AAMM extract prepared in Section 2 was obtained. (Yield: 11.3 %).

### 4. Preparation of extracts with different solvents

*Artemisia annua* from China and *Artemisia annua* from Korea were each extracted with methylene chloride, diethyl ether, ethyl acetate, acetone, or liquid carbon dioxide, and the residue remaining after each extracting solvent was removed was subjected to extraction with methanol, ethanol, or a 90% ethanol aqueous solution according to the method described above in Section 2 to thereby prepare analysis samples. Every extraction was performed according to the method described above in Section 1. The pre-extraction with liquid dioxide was performed only on the *Artemisia annua* originating from China by using a supercritical extraction method, one of the extraction methods mentioned herein, and the residue remaining after the extract obtained with liquid carbon dioxide was removed was extracted with ethanol using the method described above in Section 2 to thereby prepare samples to be used.

As a result of analysis of the extracts obtained using methanol, ethanol, or 90% ethanol aqueous solution, all the extracts exhibited similar component patterns as the AAMM extract obtained in Section 2 above.

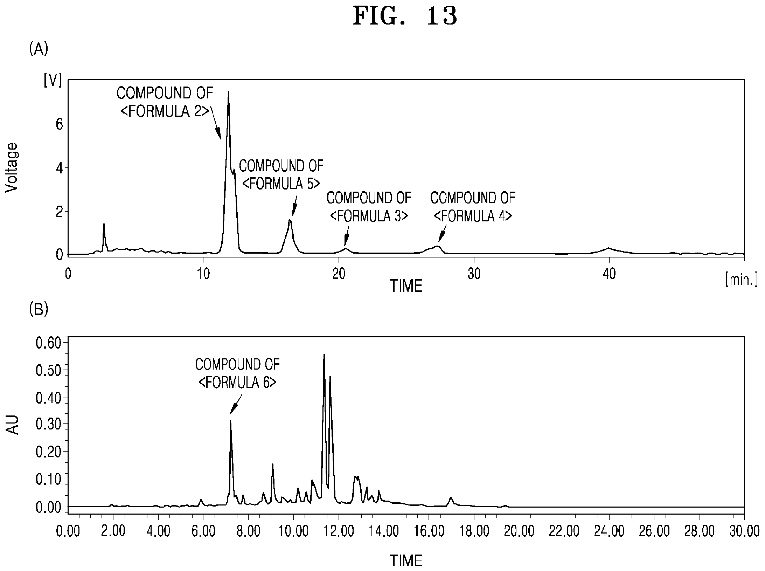

FIG. 13 illustrates HPLC results of a methanol extract of a residue remaining after an extract of *Artemisia annua* originating from China obtained with methylene chloride was removed. In FIG. 13, contents or ratios of compounds 2, 3, 4, 5, and 6 calculated based on the peak areas thereof were 4.62 %, 1.52%, 3.18%, 4.71%, and 1.00% by weight, respectively, with respect to a total weight of the extract.

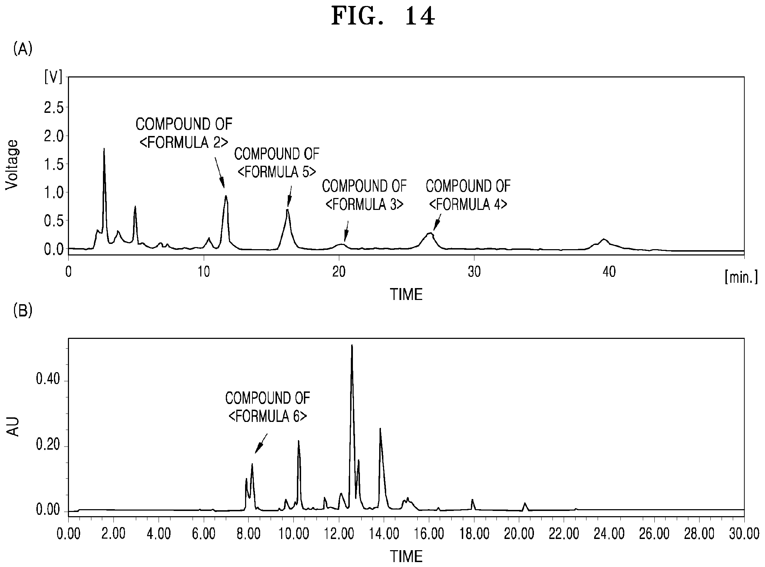

FIG. 14 illustrates HPLC results of an ethanol extract of a residue remaining after an extract of *Artemisia* annua originating from China obtained with diethyl ether was removed. In FIG. 14, contents or ratios of compounds 2, 3, 4, 5, and 6 calculated based on the peak areas thereof were 1.93 %, 0.92 %, 3.05 %, 2.75 %, and 0.57 % by weight, respectively, with respect to a total weight of the extract.

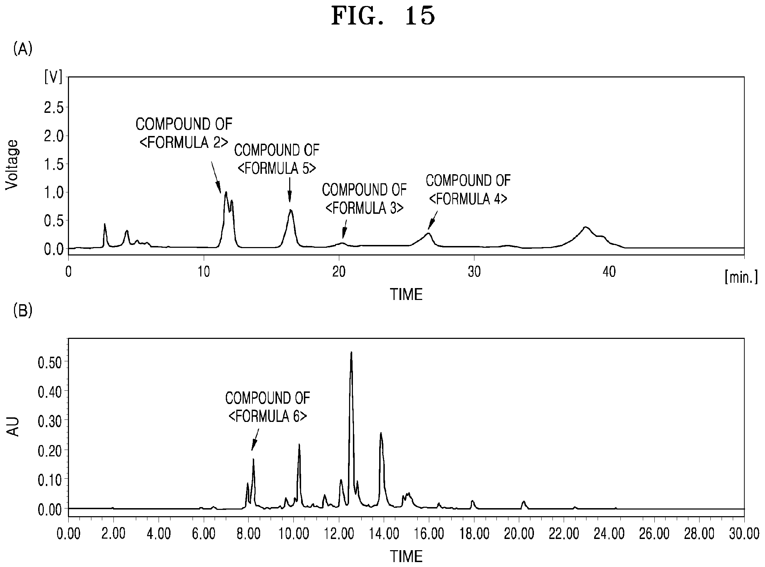

FIG. 15 illustrates HPLC results of an ethanol extract of a residue remaining after an extract of *Artemisia annua* originating from China obtained with ethyl acetate was removed. In FIG. 15, contents or ratios of compounds 2, 3, 4, 5, and 6 calculated based on the peak areas thereof were 1.14 %, 0.86 %, 2.42 %, 2.80%, and 0.83% by weight, respectively, with respect to a total weight of the extract.

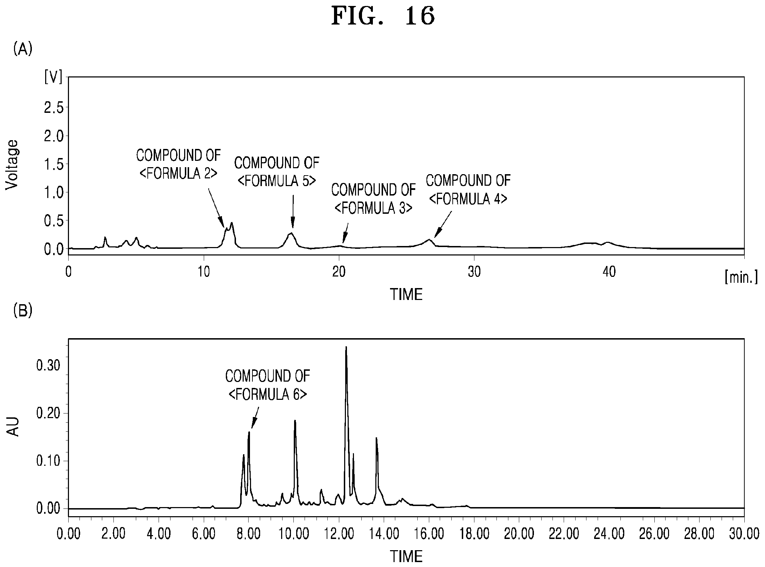

FIG. 16 illustrates HPLC results of an ethanol extract of a residue remaining after an extract of *Artemisia annua* originating from China obtained with acetone was removed. In FIG. 16, contents or ratios of compounds 2, 3, 4, 5, and 6 calculated based on the peak areas thereof were 0.66 %, 0.62 %, 2.22 %, 2.64 %, and 0.58% by weight, respectively, with respect to a total weight of the extract.

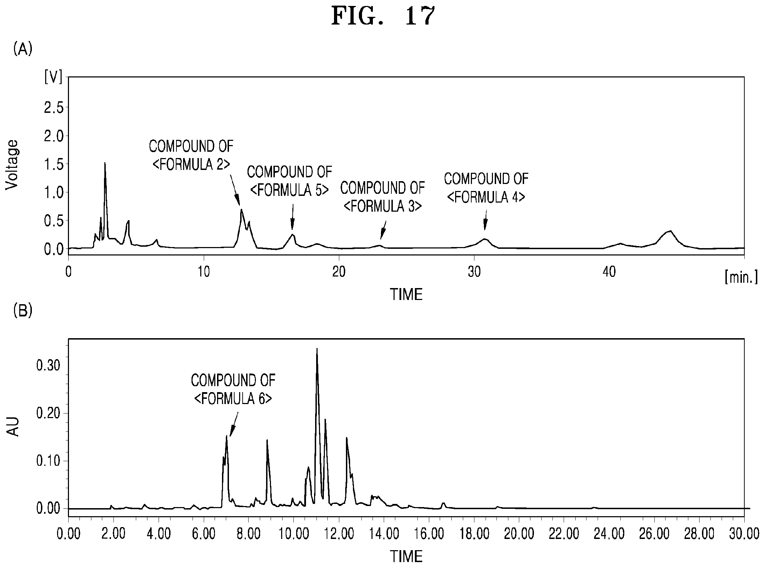

FIG. 17 illustrates HPLC results of an ethanol extract of a residue remaining after an extract of *Artemisia annua* originating from China obtained by supercritical extraction with liquid carbon dioxide was removed. In FIG. 17, contents or ratios of compounds 2, 3, 4, 5, and 6 calculated based on the peak areas thereof were 1.77 %, 0.83 %, 2.82 %, 1.55 %, and 1.12 % by weight, respectively, with respect to a total weight of the extract.

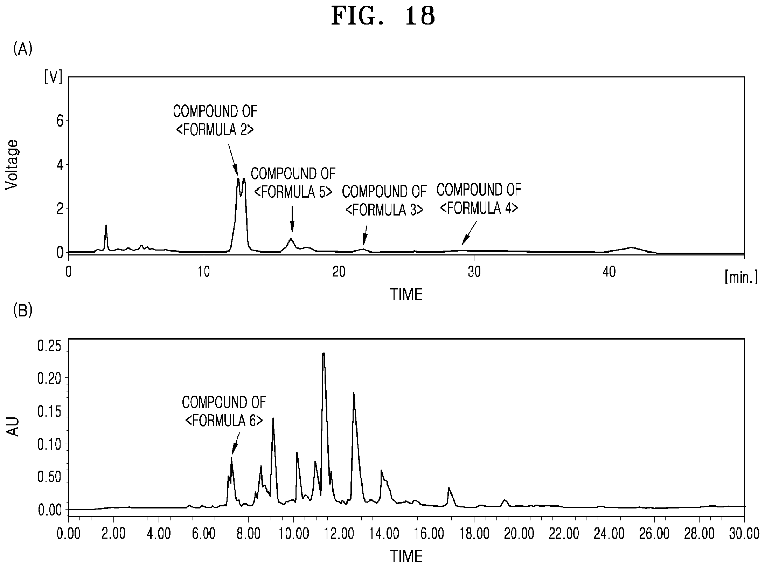

FIG. 18 illustrates HPLC results of a methanol extract of a residue remaining after an extract of *Artemisia annua* originating from Korea obtained with methylene chloride was removed. In FIG. 18, contents or ratios of compounds 2, 3, 4, 5, and 6 calculated based on the peak areas thereof were 3.43 %, 0.73 %, 1.01 %, 2.74 %, and 0.24%by weight, respectively, with respect to a total weight of the extract.

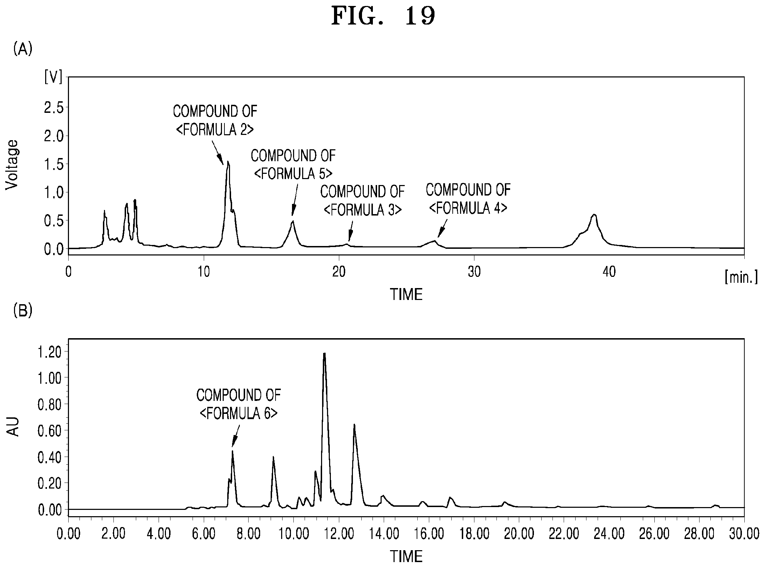

FIG. 19 illustrates HPLC results of an ethanol extract of a residue remaining after an extract of *Artemisia annua* originating from Korea obtained with diethyl ether was removed. In FIG. 19, contents or ratios of compounds 2, 3, 4, 5, and 6 calculated based on the peak areas thereof were 2.96 %, 1.14 %, 2.41 %, 2.84 %, and 1.37 % by weight, respectively, with respect to a total weight of the extract.

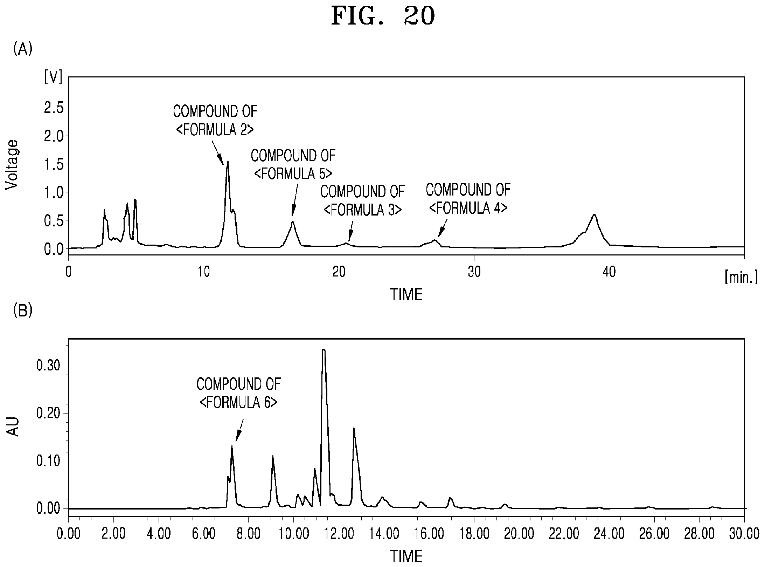

FIG. 20 illustrates HPLC results of an ethanol extract of a residue remaining after an extract of *Artemisia annua* originating from Korea obtained with ethyl acetate was removed. In FIG. 20, contents or ratios of compounds 2, 3, 4, 5, and 6 calculated based on the peak areas thereof were 1.14 %, and 0.42 %, 0.39 %, 1.24 %, and 0.46 % by weight, respectively, with respect to a total weight of the extract.

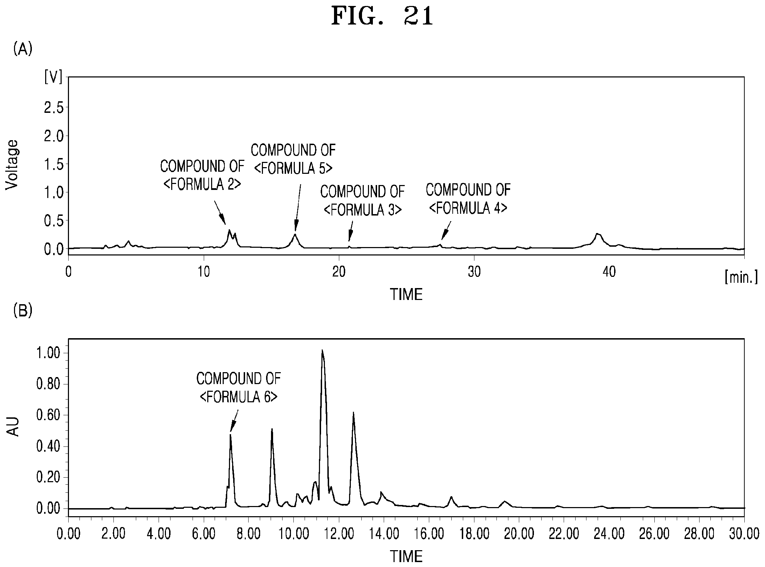

FIG. 21 illustrates HPLC results of an ethanol extract of a residue remaining after an extract of *Artemisia annua* originating from Korea obtained with acetone was removed. In FIG. 21, contents or ratios of compounds 2, 3, 4, 5, and 6 calculated based on the peak areas thereof were 1.20 %, 0.25 %, 0.93 %, 1.58 %, and 1.46 % by weight, respectively, with respect to a total weight of the extract.

According to the results of FIGS. 13 to 21, overall the extracts were found to have the contents or ratios of compounds 2, 3, 4, 5, and 6 in the ranges of about 0.66 % to about 4.62 %, about 0.25 % to about 1.53 %, about 0.39 % to about 3.18 %, about 1.24 % to about 4.71 %, and about 0.24 % to about 1.46 %, respectively.

All the resultant extracts were found to include all major active ingredients of the present disclosure, i.e., the five compounds represented by Formula 2, Formula 3, Formula 4, Formula 5, and Formula 6, respectively. The four compounds represented by Formula 2 to Formula 5 according to embodiments were analyzed using Analysis method 1 described below (panel A in FIGS. 13 to 21), and the compound of Formula 6 according to embodiments was analyzed using Analysis method 2 described below (panel B in FIGS. 13 to 21). The extracts obtained by extraction with different solvents as described above were found to include similar components as those of the ethanol extracts obtained from the residue after the extraction with dichloromethane. The extracts also had a similar effect of ameliorating inflammatory skin, like an AAMM extract and fractions thereof described below in test examples.

### Analysis method 1

Analysis equipment: HPLC (YL9100 series, available from YOUNG IN
Detector: ELSD (Temp: 50 °C, Gas: 2.5 In) (GmbH, ZAM 3000)
Analysis conditions: 1 mL/min, 87% A (MeCN); 13% B (D.W), isocratic 50 min
Column: YMC NH₂ (5 µm, 150 × 4.6 mm)
Column temperature: 30 °C
Injection volume: 10 µL

### Analysis method 2

Analysis equipment: HPLC (Waters)
Detector: 2996 PDA detector (254 nm) (Waters)
Analysis conditions: 1 mL/min, A (0.02% TFA in MeCN); B (0.02% TFA in D.W), 0 min:
   10 % A -> 30 min: 100 % A
   Column: Phenomenex Luna C18(2) (5 µm, 150 × 4.6 mm)
   Column temperature: room temperature
   Injection volume: 10 µL

### Example 2: Preparation of fractions of AAMM extract

16 g of the AAMM extract described above in Section 2 of Example 1 was fractionated using ethanol, water, or a mixed solvent of ethanol and water, to thereby prepare fractions of the AAMM extract. In particular, 1 L of ethanol and mixtures of ethanol and water in a ratio by volume of about 2:8, about 4:6, about 6:4, and about 10:0, respectively, were used as fractionating solvents.

That is, the AAMM extract was contacted with part of a mixture of ethanol and water used as a fractionating solvent, then loaded over 100 g of octadecyl-silica (ODS) resin used as a fixed phase, and then eluted by chromatography with each of the solvents to obtain four fractions ['AAMM-fraction 1' (a 2:8 mixture of ethanol and water as fractionating solvent, 9.6 g), 'AAMM-fraction 2' (a 4:6 mixture of ethanol and water, 2.4 g), 'AAMM-fraction 3' (a 6:4 mixture of ethanol and water as fractionating solvent, 1.3 g), and 'AAMM-fraction 4' (a 10:0 mixture of ethanol and water, 1.5 g)].

Efficacy of each fraction was evaluated using a method described below in Test Example 2, and AAMM-fraction 1 and AAMM-fraction 4 having relatively good efficacy were mixed in a ratio of about 9:1 according to their yields to thereby prepare a 'AAMM-fraction 1+4' sample.

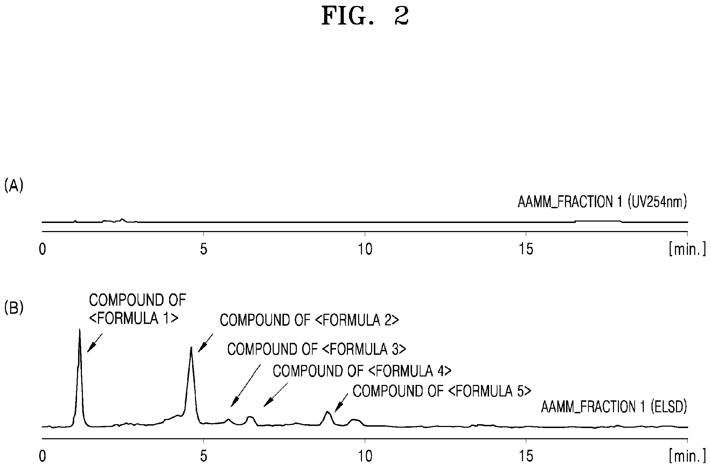

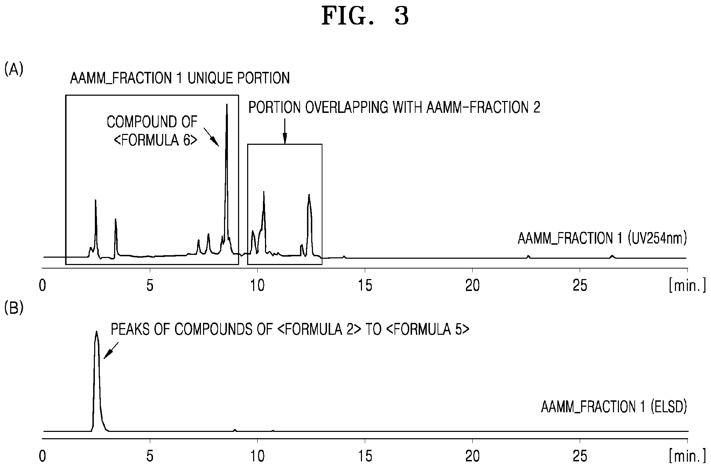

FIGS. 2 and 3 illustrate HPLC results of AAMM-fraction 1. The HPLC conditions of FIG. 2 are as Condition 1 below, and those of FIG. 3 are as Condition 2 below. The results of (A) in FIGS. 2 and 3 were obtained using a UV detector (254 nm), and the results of FIGS. 2B and 3B were obtained using an ELSD detector.

Referring to (B) in FIG. 2, as the results of the HPLC under Condition 1, the compounds represented by Formulae 1 to 5 were detected as major peaks. Referring to FIGS. 3, as the results of the HPLC under conditions 2, chlorogenic acid represented by Formula 6 was detected as a major peak.

### Condition 1:

Equipment used: YL 9100 HPLC (available from YOUNGIN)
Column: NH₂ HPLC column (4.6x250 mm, 5 µm)
Mobile phase composition: Acetonitrile and water in a volume ratio of 80:20 (20 minutes)
Flow rate of mobile phase: 1.0 mL/min
Detector: UV detector - 254 nm (FIG. 2A), and ELSD detector ((B) in FIG. 2)

### Condition 2:

Equipment used: YL 9100 HPLC (available from YOUNGIN)
Column: RP C-18 HPLC column (4.6x150 mm, 5 µm)
Solvent:
   (a) Acetonitrile including 0.02 % of trifluoroacetic acid (TFA)
   (b) Water including 0.02 % of TFA
Mobile phase composition: Elution starts with a 10:90 ratio by volume of solvent (a) to solvent (b) at 0 minutes, then with an increasing ratio of volume of solvent (a) from 10 % to 100 % at 0 minutes to 30 minutes, and then with 100 % by volume of solvent (a) at 30 minutes to 37 minutes
Flow rate of mobile phase: 1.0 mL/min
Detector: UV detector - 254 nm ((A) in FIG. 3), and ELSD detector ((B) in FIG. 3)

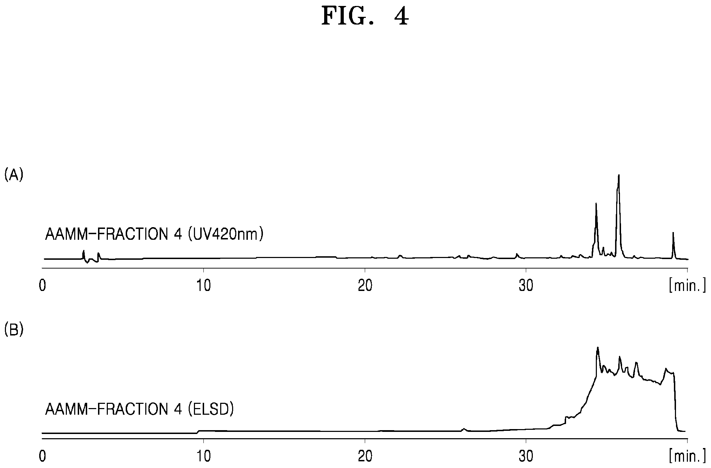

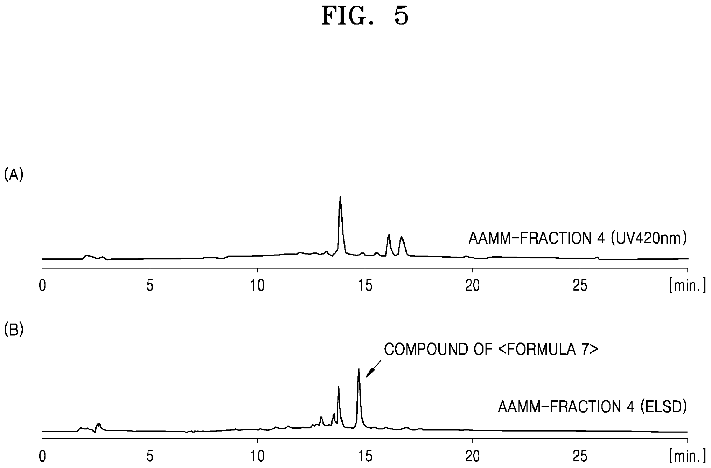

FIGS. 4 and 5 illustrate HPLC results of AAMM-fraction4. The HPLC conditions of FIG. 4 are as Condition 3 below, and those of FIG. 5 are as Condition 4 below. The results of (A) in FIGS. 4 and 5 were obtained using a UV detector (420 nm), and the results of (B) in FIGS. 4 and 5 were obtained using an ELSD detector.

Referring to (A) in FIG. 4, components estimated to be carotenoids or chlorophyll were detected as major peaks by the UV detector (detection wavelength: 420 nm). Referring to (B) in FIG. 5, α-linolenic acid represented by Formula 7 were detected as a major unsaturated fatty acid component by the ELSD detector, along with other similar fatty acid components as major peaks.

### Condition 3:

Same conditions as Condition 2, except for the following condition:
Detector: UV detector - 420 nm ((A) in FIG. 4), and ELSD detector ((B) in FIG. 4).

### Condition 4:

Equipment used: YL 9100 HPLC (available from YOUNGIN)
column: RP C-18 HPLC column (4.6x150 mm, 5 µm)
Solvent:
   (a) Methanol including 0.02 % of TFA
   (b) Water including 0.02 % of TFA
Mobile phase composition: Elution starts with an 80:20 ratio by volume of solvent (a) to solvent (b) at 0 minutes and then increasing ratio of volume of solvent (a) from 80 % to 100 % from 0 minutes to 10 minutes, and then with 100 % by volume of solvent (a) at 10 minutes to 30 minutes.
Flow rate of mobile phase: 1.0 mL/min
Detector: UV detector - 420 nm ((A) in FIG. 5), and ELSD detector ((B) in FIG. 5)

### Example 3: Isolation and identification of compounds as major compound

### 1. Isolation and identification of components contained in AAMM-fraction 1

The AAMM-fraction 1 of Example 2 was concentrated under reduced pressure, and then separated into the subfractions by HPLC according to Separation method 1 below.

As a result, ornithine, pinitol, muco-inositol, chiro-inositol, and betaine were separated. From 100 mg of AAMM-fraction 1 on a dry weight basis, about 3 mg of ornithine, about 29 mg of pinitol, about 4 mg of muco-inositol, about 7 mg of chiro-inositol, and about 3 mg of betaine were obtained. In addition, fructose was separated with a yield of about 15 % on a dry weight basis of the AAMM-fraction 1.

### Separation method 1

Equipment used: Waters 515 pump
Column: YMC NH₂ HPLC column (10x250 mm, 10 µm)
Solvent:
   (a) Acetonitrile
   (b) Water
Mobile phase composition: Elution starts with a 80:20 ratio by volume of solvent (a) to solvent (b) at 0 minutes, then with the same ratio of from 6 minutes to 60 minute.
Flow rate of mobile phase: 4.0 mL/min
Detector: RI

The AAMM-fraction 1 was separated into subfractions by HPLC according to Separation method 2 below. As a result, chlorogenic acid according to embodiments were separated with a yield of 7 mg from 100 mg of the AAMM-fraction 1.

### Separation method 2

Equipment used: YMC LC-Forte R
Column: Phenomenex Luna C-18 RP HPLC column (21.2x250 mm, 10 µm) Solvent:
   (a) Acetonitrile containing 0.02 % of TFA
   (b) Water containing 0.02 % of TFA
Mobile phase composition: Elution starts with a 10:90 ratio by volume of solvent (a) to solvent (b) at 0 minutes, then with an increasing ratio of volume of solvent (a) from 10 % to 40 % at 0 minutes to 40 minutes
Flow rate of mobile phase: 20.0 mL/min
Detector: UV detector - 254 nm, and ELSD detector

The separated compounds were analyzed by nuclear magnetic resonance (NMR) and mass spectrometry (MS) to obtain data, which were then compared with disclosed reference documents to thereby identify structures thereof.

In particular, the molecular weight of ornithine was determined to be about 132 by mass spectrometry (MS) with an Agilent 1100 high-performance liquid chromatography-electrospray ionization mass spectrometry (HPLC-ESI-MS) system. The structure of ornitrine was estimated to be Formula 1 by ¹H and ¹³C-NMR spectrum analysis with a Varian 500 MHz NMR. The NMR data ware compared with data of a disclosed document (J.C. MacDonald et al. Can. J. Chem. 1976, 54, 1126-1133) to thereby identify the structure thereof.

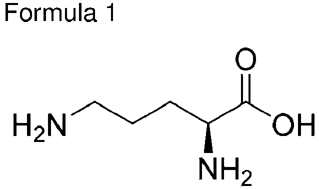

White powder; (molecular formula) C₅H₁₂N₂O₂, ESI-MS: m/z 132 [M-H]⁻;
¹H NMR (500 MHz, D₂O): δ 3.94(1H, dd, J = 9.0, 6.5 Hz), 3.23(1H, dt, J = 11.5, 7.0 Hz), 3.14(1H, dt, J = 11.5, 7.0 Hz), 2.16(1H, m), 1.89 (1H, m), 1.82 (2H, m) ¹³C NMR (125 MHz, D₂O) : δ 174.6, 61.1, 45.9, 28.8, 23.6.

The molecular weight of pinitol was determined to be about 194 by mass spectrometry with an Agilent 1100 high-performance liquid chromatography-electrospray ionization mass spectrometry (HPLC-ESI-MS) system. The structure of pinitol was estimated to be Formula 2 by ¹H and ¹³C-NMR spectrum analysis with a Varian 500 MHz NMR. The NMR data ware compared with data of disclosed documents (M. Della greca et al. Chem. Biodivers. 2007, 4, 118-128; C. Sridhar et al. Ind. J. Pharm. Sci. 2006, 68, 111-114) to thereby identify the structure thereof. The stereochemistry of Formula 2 is a relative stereochemistry.

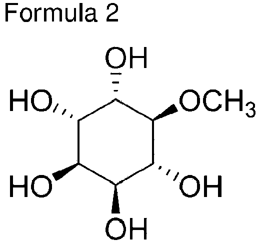

White powder; (molecular weight) C₇H₁₄O₆, ESI-MS: m/z 193 [M-H]⁻;
¹H NMR (500 MHz, D₂O*): δ 3.28(1H, t, J = 9.5 Hz), 3.54(3H, s), 3.59(1H, t, J = 9.5 Hz), 3.70(1H, dd, J = 9.5, 3.0 Hz), 3.75 (1H, dd, J = 9.5, 3.0 Hz), 3.95(1H, m), 3.94 (1H, m), *D₂O δ4.79; ¹³C NMR (125 MHz, D₂O): δ 82.6, 72.0, 71.5, 71.3, 70.3, 69.7, 59.6.

The molecular weight of muco-inositol was determined to be about 180 by mass spectrometry with an Agilent 1100 high-performance liquid chromatography-electrospray ionization mass spectrometry (HPLC-ESI-MS) system. The structure of muco-inositol was estimated to be an inositol derivative having a symmetric structure represented by Formula 3. The NMR data of muco-inositol were similar to NMR data of a disclosed document (S.J. An gyal and L. Odier, Carbohydrate Res. 1982, 100, 43-54), confirming the structure. The stereochemistry of Formula 3 is a relative stereochemistry.

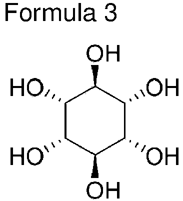

White powder; (molecular weight) C₆H₁₂O₆, ESI-MS: m/z 179 [M-H]⁻;
¹H NMR (500 MHz, D₂O*): δ 3.95 (2H, br t, J = 6.0 Hz), 3.82 (4H, br d, J = 6.0 Hz), *D₂O δ4.79; ¹³C NMR (125 MHz, D₂O): δ 72.1, 70.0.

The molecular weight of chiro-inositol was determined to be about 180 by mass spectrometry with an Agilent 1100 high-performance liquid chromatography-electrospray ionization mass spectrometry (HPLC-ESI-MS) system. The structure of chiro-inositol was estimated to be inositol having a structure represented by Formula 4 by ¹H and ¹³C-NMR spectrum analysis with a Varian 500 MHz NMR. The NMR data of chiro-inositol ware similar to NMR data of a disclosed document (R. Abraham et al. Ma gn. Reson. Chem. 2005, 43, 611-624), confirming the structure. The stereochemistry of Formula 4 is a relative stereochemistry.

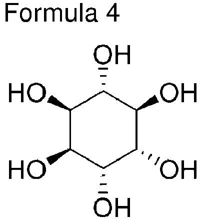

White powder; (molecular weight) C₆H₁₂O₆, ESI-MS: m/z 179 [M-H];
¹H NMR (500 MHz, D₂O*): δ 3.96 (1H, br t, J = 2.5 Hz), 3.96(1H, br t, J = 2.5 Hz), 3.69(1H, br t, J = 2.5 Hz), 3.68(1H, br t, J = 2.5 Hz), 3.53(1H, t, J = 6.5 Hz), 3.51 (1H, t, J = 6.5 Hz). *D₂O δ4.79; ¹³C NMR (125 MHz, D₂O): δ 72.6, 71.5, 70.3.

The structure of betaine was estimated to be Formula 5 by ¹H and ¹³C-NMR spectrum analysis with a Varian 500 MHz NMR. The NMR data ware compared with data of disclosed documents (W. Gronwald et al. Anal. Chem. 2008, 80, 9288-9297; D. godzisz et al. J. Mol. Struct. 2002, 606, 123-137) to identify the structure thereof.

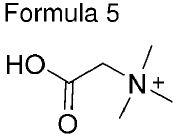

White powder; ¹H NMR (500 MHz, D₂O*): δ 3.85 (2H, s), 3.21 (6H, s) *D₂O δ4.79; ¹³C NMR (125 MHz, D₂O): δ 169.1, [65.97, 95.94, 95.92], [53.13, 53.16, 53.19].

The molecular weight of chlorogenic acid was determined to be about 355 by mass spectrometry with an Agilent 1100 high-performance liquid chromatography-electrospray ionization mass spectrometry (HPLC-ESI-MS) system. The structure of chlorogenic acid was estimated to be Formula 6 by ¹H-NMR spectrum analysis with a Varian 500 MHz NMR. The NMR data were compared with data of a disclosed document (T. Han et al. Chem. Nat. Comp. 2006, 42, 597-570) to thereby identify the structure thereof.

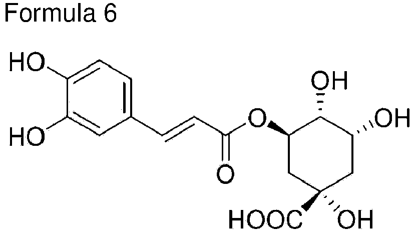

White powder; (molecular weight) C₁₆H₁₈O₉; ESI-MS: m/z 355 [M+H]⁺;
¹H NMR (500 MHz, CD₃OD): δ 7.56 (1H, d, J = 16.0 Hz), 7.05 (1H, d, J = 1.5 Hz), 6.95 (1H, dd, J = 8.0, 1.5 Hz), 6.78 (1H, d, J = 8.0 Hz), 6.27 (1H, d, J = 16.0 Hz), 5.33 (1H, br td, J = 9.5, 3.0 Hz), 4.17 (1H, br m), 3.73 (1H, br dd, J = 9.5, 2.5 Hz), 2.18 (2H, br m), 2.04 (2H, br m).

### 2. Isolation and identification of major components of AAMM-fraction 4

The AAMM-fraction 1 of Example 2 was concentrated under reduced pressure and then fractionated into subfractions by HPLC according to the following separation method.

As a result, α-linolenic acid according to embodiments was separated. About 25 mg of α-linolenic acid was obtained from 100 mg of the AAMM-fraction 4. During the separation process of α-linolenic acid, linoleic acid and palmitic acid were additionally separated with a yield of about 10% on a dry weight basis of the AAMM-fraction 4, respectively.

### Separation method

Equipment used: YMC LC-Forte R
Column: Phenomenex Luna C-18 RP HPLC column (21.2x250 mm, 10 µm)
Solvent:
   (a) Methanol containing 0.02 % of TFA
   (b) Water containing 0.02 % of TFA
Mobile phase composition: Elution starts with a 90:10 ratio by volume of solvent (a) to solvent (b) at 0 minutes, then with an increasing ratio of volume of solvent (a) from 90 % to 100 % at 0 minutes to 10 minutes, and then with the same ratio of solvent (a) at 10 minutes to 40 minutes.
Flow rate of mobile phase: 20.0 ml/min
Detector: UV detector - 210 nm and ELSD detector

The separated compounds were analyzed by gas chromatography-mass spectrometry (GC-MS) and NMR to obtain data, and then compared with disclosed reference documents to thereby identify structures thereof.

In particular, the molecular weight of α-linolenic acid was determined to be about 278 with a GC-MS analyzer. The structure of α-linolenic acid was estimated to be Formula 7 by GC-MS Database searching and ¹H-NMR spectrum analysis with a Varian 500 MHz NMR.

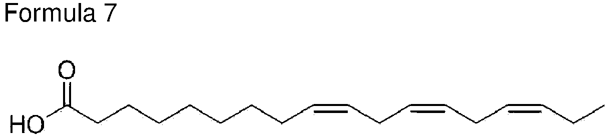

### α-linolenic acid

Colorless; (molecular formula) C₁₈H₃₀O₂; ESI-MS: m/z 301 [M+Na]⁺;
¹H NMR (500 MHz, CDCl₃): δ 0.97 (3H, t, J = 7.0 Hz), 1.30-1.38 (8H, br m), 1.62 (2H, m), 2.06 (4H, m), 2.35 (2H, t, J = 7.5 Hz), 2.81 (4H, br m), 5.31-5.42 (6H, m).

### Example 4: Preparation of mixtures of compounds of Formulae 1 to 7

To identify active ingredients of the *Artemisia annua* extract prepared in Example 1 and the *Artemisia annua* fractions prepared in Example 2, some or all of the single compounds separated in Example 3 were mixed again in a similar composition as to the yields from the parent fractions.

In particular, a mixture of at least four compounds selected from the six compounds represented by Formulae 1 to 6 separated in Section 1 of Example 3, or a composition in which α-linolenic acid, linoleic acid and palmitic acid separated in Section 2 of Example 3 were mixed in an equal amount was prepared. In addition, to the mixture of at least four compounds selected from the six compounds represented by Formulae 1 to 6, at least one compounds of α-linolenic acid or a similar unsaturated fatty acid or vegetable saturated fatty acid was added to prepare a mixture. The components and ratios thereof in the prepared mixtures are represented in Table 1.

Table 1 shows the components and composition ratios of the mixtures of the compounds separated from AAMM-fraction 1 and AAMM-fraction 4.

**[Table 1]**

| Single compounds | | Sample codes of mixtures and mixed composition ratios of single compounds (by weight) | | | | | | | | |
|---|---|---|---|---|---|---|---|---|---|---|
| Names | Codes | AA-M2 | AA-M3 | AA-M4 | AA-M5 | AA-M6 | AA-M7 | AA-M8 | AA-M9 | AA-M10 |
| Ornithine | AA-M1-A | 1 | 1 | - | - | 1 | 1 | 1 | 1 | 1 |
| Pinitol | AA-M1-B | 8 | 8 | - | - | 8 | 8 | 8 | 8 | 8 |
| Muco-inositol | AA-M1-E | 1 | 1 | - | - | - | - | - | 1 | 1 |
| Chiro-inositol | AA-M1-D | 2 | 2 | - | - | 2 | 2 | 2 | 2 | 2 |
| Betaine | AA-M1-F | 4 | 4 | - | - | 4 | 4 | 4 | 4 | 4 |
| Chlorogenic acid | AA-M1-J | - | 2 | - | 8 | - | 2 | - | 2 | 2 |
| Fructose | AA-M1-C | - | - | - | - | - | - | - | - | - |
| α-linolenic acid | AA-M1-H | - | - | 1 | 1 | - | - | 1.8 | 2.7 | 1.8 |
| Linoleic acid | AA-M1-G | - | - | 1 | 1 | - | 4 | 1.8 | 2.7 | 1.8 |
| Palmitic acid | AA-M1-I | - | - | 1 | - | - | - | - | - | 1.8 |

### Test Example 1: Dermatitis relieving effects of two extracts (AAM and AAMM) prepared in Example 1

To investigate dermatitis relieving effects of *Artemisia annua* dichloromethane extract (AAM) prepared in Example 1, and *Artemisia annua* ethanol extract (AAMM) prepared from the residue remaining after the extraction with dichloromethane in Example 1, the present inventors purchased NC/Nga atopic dermatitis mouse models (6-week aged males having a body weight of about 30 g per subject, available from Central Laboratory Animal Inc.), which were housed in an air flow-through sterile test bench and freely fed with standard solid feeds for 1 week. For use in experiments, all test reagents were dissolved in a small amount of dimethyl sulfoxide (DMSO) and diluted with olive oil until a final concentration of 0.2 % was reached. On the 1^{st} day immediately after the back hair of each mouse was removed, 0.15 % of 1-fluoro-2,4-dinitrobenzene (DNFB) was applied onto the hair-removed back. Then, 0.15 % of DNFB was applied again onto the back on the 5^{th} day, and 0.2 % of DNFB was applied onto the back on the 9^{th} day to induce skin itching and atopic dermatitis caused by DNFB, followed by treatment with test drugs. In particular, 2 % of DNFB and test drugs were applied on the 11^{th} day, 13^{th} day, 15^{th} day, and 17^{th} day (i.e., four times in total). Then, the states of the DNFB-induced inflammation group, the AAM treatment group, and the AAMM treatment group were observed by positron emission tomography-computed tomography (PET-CT) imaging with¹⁸F-fluorodeoxy glucose (FDG) tracer and tissue staining microscopy.

To obtain PET-CT images, all the mice were fasted with access only to water for 12 hours. ¹⁸F-fluorodeoxyglucose (¹⁸F-FDG) of 37 MBq (1.0 mCi) was administered via the tail vein of the mice, and after 60 minutes, each mouse was then scanned by emission scanning with a small animal PET scanner for about 22 minutes.

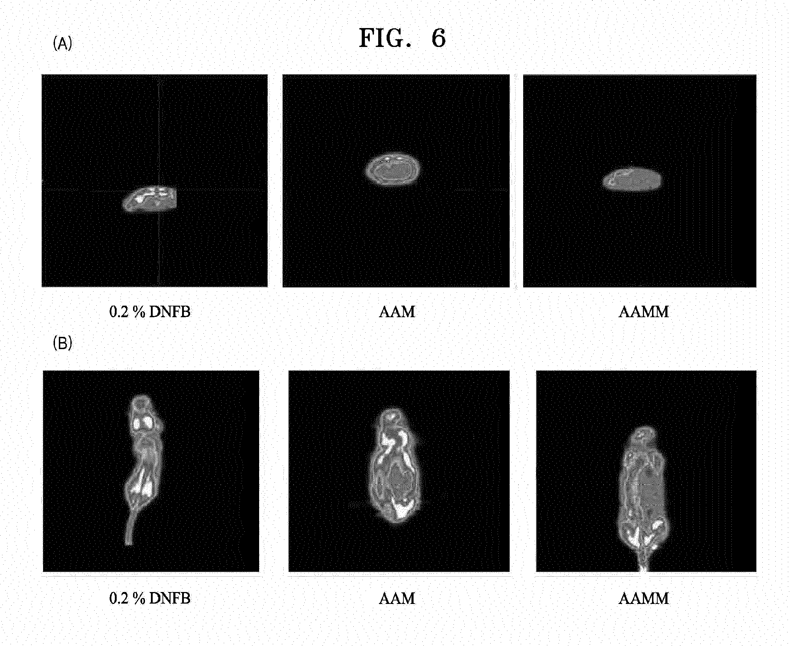

FIG. 6 shows ¹⁸F-FDG PET-CT images of the mice of which inflammatory sites were treated with the *Artemisia annua* dichloromethane extract (AAM) and the *Artemisia annua* ethanol extract (AAMM) obtained from the residue remaining after dichloromethane extraction. FIG. 6A illustrates transaxial plane images, and FIG. 6B illustrates coronal plane images.

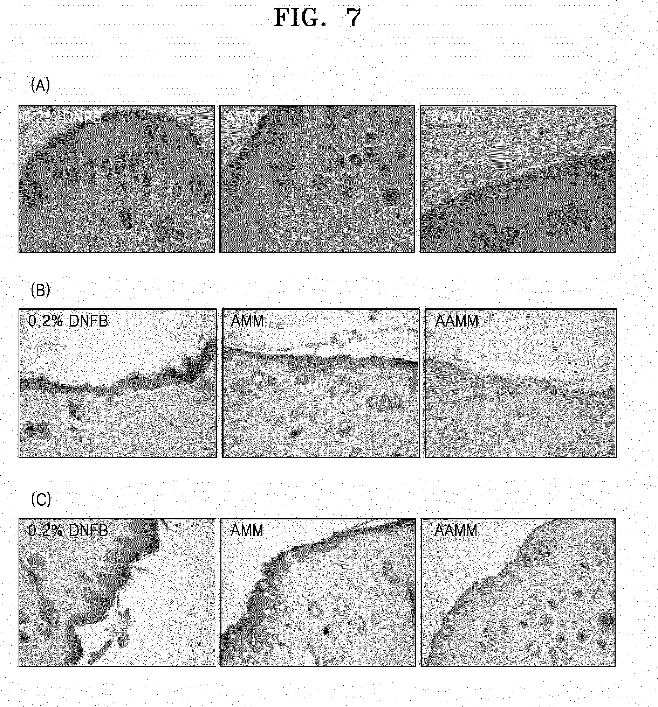

FIG. 7 illustrates microscopic images showing changes in tissues after inflammatory sites of the mice were treated with AAM and AAMM. In particular, FIG. 7A illustrates images (x200 magnification) obtained by histopathology microscopy with hematoxylin and eosin (H&E) staining, FIG. 7B illustrates images (x200 magnification) obtained by immunohistochemical microscopy using rabbit monoclonal primary keratin 10 polyclonal antibody, and FIG. 7C illustrates images (x200 magnification) obtained by immunohistochemical microscopy using rabbit monoclonal primary substance P.

PET-CT imaging conditions:

| | |
|---|---|
| Field-of-view (FOV) | 38mm |
| Energy window | 350 keV to 650 keV |
| Algorithm/reconstruct | OSEM 2D/iteratioin (2 times), subset: 8 |
| Matrix size (mm) | 128 x 128 |
| Scan Time (min) | 22 min |
| P.I time (min) | 60 min |
| RI/Activity (uCi) | ¹⁸F/ 1mCi |
| Focal spot size | 50 µm |
| X-ray detector pixel | 2,048 x 3,072 |
| 80 kV, 500 µA, 3600 of 200 projections rotation, Cone-beam Filter | |

After the PET-CT scanning, skin cells of the mice were taken and fixed with 4 % paraformaldehyde (pH 7.4). The tissue samples were sectioned to a thickness of about 5 um, stained with hematoxylin and eosin (H&E), and then observed using optical microscopy.

In order to observe changes in tissues by immunohistochemistry, the 10-um-thick tissue sections were incubated in a 3% fetal bovine serum in phosphate buffered saline (PBS) for about 1 hour, and then incubated overnight after rabbit monoclonal primary keratin 10 polyclonal antibody (1:100 dilution, Covance, USA) or rabbit monoclonal primary substance P antibody (1:100 dilution, Abcam, USA) was added thereto. Each sample was washed three times with PBS, incubated with Vectastain ABC reagents, and reacted with 3,3-diaminobenzidine tetrahydrochloride (DAB) (available from Vector laboratories, USA).

Table 2 shows the radio-isotope ¹⁸F-FDG uptake densities (UD) in drug treatment sites.

**[Table 2]**

| Drug treatment groups | 0.2% DNFB | AAM (0.2%) | AAMM (0.2%) |
|---|---|---|---|
| UD | 9.71 | 2.40 | 0.17 |

Referring to Table 2, FIG. 6, and FIG. 7, the *Artemisia* ethanol extract (AAMM), corresponding to an extract obtained by the extraction with dichloromethane from which sesquiterpene lactone components such as artemisinin and arteenuanine B were removed, was found to have a significant effect of relieving DNFB-induced atopic dermatitis and itching.

Referring to FIG. 6, in the DNFB-induced inflammation group (0.2 % DNFB) and the AAM treatment group (0.2 %), inflammatory sites appeared red (peripheral regions of bright areas) due to accumulation of ¹⁸F-FDG in cells. Meanwhile, in the AAMM treatment group, nearly no cell inflammatory reactions occurred at drug application sites, which appeared only blue (regions except for the red regions), and the red color due to the accumulation of ¹⁸F-FDG in cells was not observed. ¹⁸F-FDG PET is widely used for the differential diagnosis of malignant tumors and inflammation and the evaluation of improvement or not and responses to treatment by imaging cells of inflammatory tissues caused due to hypermetabolism of glucose or malignant tumor cells against normal cells. Such increased accumulation of FDG in the cells of inflammatory or malignant tumor tissues occurs because FDG, similarly to glucose, is transported into cells by a glucose transport protein and also phosphorylated by hexokinase in cells, but unlike glucose, phosphorylated FDG, i.e., FDG-6-phosphase, accumulates because it is no longer able to be metabolized. FDG-6-phosphate as a very strong polar substance fails to diffuse back out of the cells, resulting in high accumulation of FDG.

Referring to (A) in FIG. 7, as a result of observation of the inflammatory tissue sections after H&E staining, in the DNFB-induced inflammation group (0.2 % DNFB) and the AAM treatment group (0.2 %), a large number of elongated prickle cell layers or circular inflammatory reaction cells, appearing as dark blue (darker shade), were observed in the epidermis. Meanwhile, in the AAMM treatment group (0.2 %), comparatively clear epidermal tissues were observed.

In FIG. 7, (B) illustrates images obtained by selective staining of keratinus keratin in the inflammatory tissues. In the DNFB-induced inflammation group (0.2 % DNFB) and the AAM treatment group (0.2 %), a large amount of keratin stained with a dark brown layer (darker color) was observed on the epidermal surface. However, in the AAMM treatment group (0.2 %), a very thin keratin layer was observed on the epidermal surface, like normal skin tissues.

In FIG. 7, (C) illustrates images obtained by selective staining of the substance P known to cause inflammatory reaction and itching at the inflammatory tissues. In the DNFB-induced inflammation group (0.2 % DNFB) and the AAM treatment group (0.2 %), a large amount of the substance P stained with a dark brown layer (darker color) was observed on the epidermal surface. Meanwhile, in the AAMM treatment group (0.2 %), nearly no or a very small amount of the substance P was observed as in normal skin tissues.

### Test Example 2: Dermatitis relieving effects of fractions prepared in Example 2

To compare dermatitis relieving effects of the *Artemisia annua* ethanol extract (AAMM) prepared from the residue remaining after the extraction with dichloromethane in Example 1 and the four fractions, i.e., AAMM-fraction1, AAMM-fraction 2, AAMM-fraction 3, and AAMM-fraction 4, prepared from the AAMM extract in Example 2, with the efficacy of dexamethasone as a steroidal anti-inflammatory agent, the present inventors purchased NC/Nga atopic dermatitis mouse models (6-week aged males having a body weight of about 30 g per subject, available from Central Laboratory Animal Inc.), which were accommodated in an air flow-through sterile test bench and freely fed with standard solid feeds for 1 week. Inflammation induction and drug treatment were carried out in the same manner as in Test Example 1. For use in experiments, all test reagents were dissolved in a small amount of dimethyl sulfoxide (DMSO) and diluted with olive oil until a final concentration of 0.2 % was reached. Test animal models were classified into a control group to which only olive oil was applied instead of drugs, a negative control group (DNFB) in which dermatitis was induced by treatment with 0.2 % of DNFB, a positive control group (dexamethasone) in which 0.2 % of dexamethasone was applied onto dermatitis-induced sites, a comparative group (AAMM) to which 0.2 % of AAMM was applied, and drug treatment groups in which 0.2 % of the above-identified four fractions were respectively applied to inflammation-induced skin sites. Drug treatment with 0.2 % of DNFB, the comparative drug, and each test drug was performed on the 11^{th} day, 13^{th} day, 15^{th} day, and 17^{th} day (i.e., four times in total in each group). Then, skin cells of the mice were taken and fixed with 4 % paraformaldehyde (pH 7.4) in the same manner as in Example 1. The tissue samples were sectioned to a thickness of about 5 um, stained with hematoxylin and eosin (H&E), and then observed using optical microscopy.

The level of expression and distribution of collagen were microscopically observed by using picrosirius red (PSR) to stain red the collagen in the tissue sections of inflammation-induced and drug treatment sites. To investigate the degrees of inflammation and improvement in each experimental group, the states of the control group, the DNFB treatment group, the dexamethasone treatment group, the AAMM treatment group, and each fraction treatment group were compared with the DNFB-induced inflammation group by image comparison.

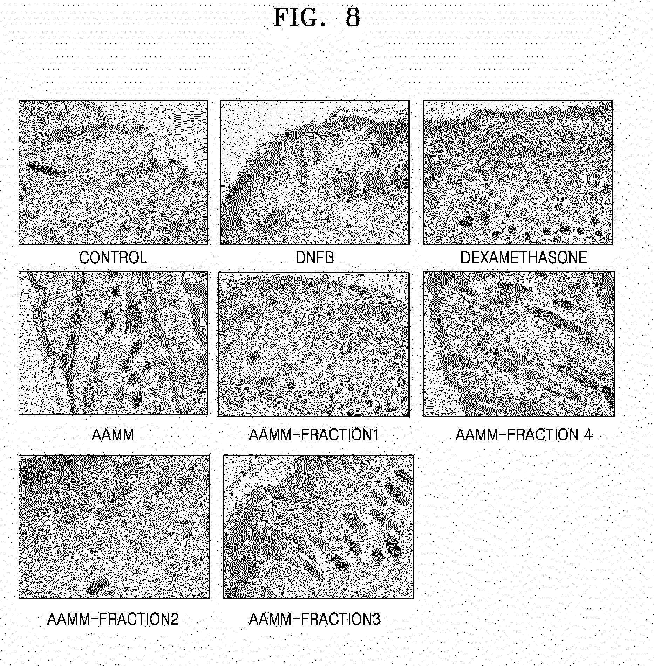

FIG. 8 illustrates histophatological microscope images after H&E staining showing changes in tissues after the treatment of inflammatory sites in mice with DNFB, dexamethasone, AAMM, AAMM-fraction 1, AAMM-fraction 2, AAMM-fraction 3, and AAMM-fraction 4 (x200 magnification).

As shown in FIG. 8, as a result of the microscopic observation of the inflammatory tissue sections after H&E staining, in the DNFB-induced inflammation group (0.2 % DNFB), as compared with normal tissues (control group), red-stained thick keratin layer (darker color layer) and elongated acathosis on the epidermal surface, and a large number of inflammatory cells (many successive dots under the stained keratin layer, and population of large circular bodies) stained in darker blue with a collapsed epidermal layer were observed. Meanwhile, in the dexamethasone treatment group and the AAMM treatment group, thin distribution of keratin on the epidermal surface and a very small number of acanthosis and inflammatory cells were observed, indicating greatly relieved inflammatory symptoms. In the AAMM-fraction 1 or AAMM-fraction 4 treatment groups, elongated acanthosis stained in dark blue were observed on the epidermis, indicating weaker efficacy than the dexamethasone treatment group and the AAMM treatment group. However, comparatively thin distribution of keratin and nearly no inflammatory cell supported relatively good efficacy, as compared with the AAMM-fraction 2 or AAMM-fraction 3 treatment group. In the AAMM-fraction 2 treatment group, a large number of inflammatory responses were observed. In the AAMM-fraction 3 treatment group, comparatively thin distribution of keratin and less acanthosis were observed, indicating reduced inflammatory responses. However, severe breakdown of the collagen layer indicated slightly weak efficacy.

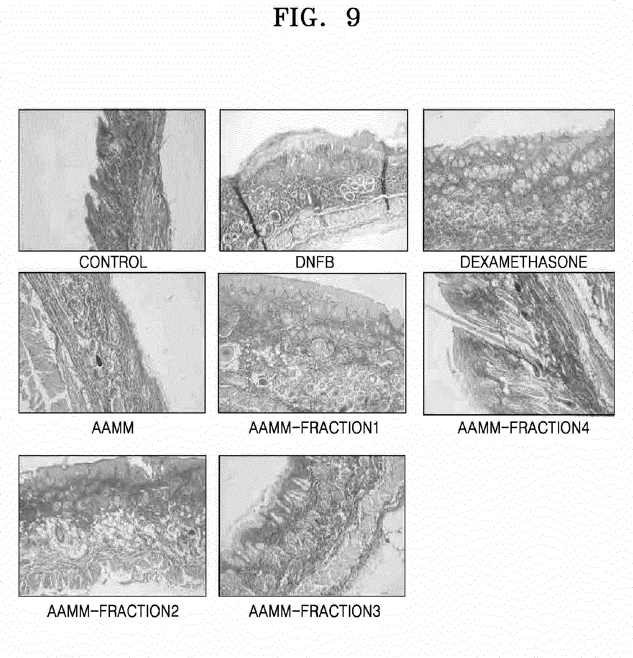

FIG. 9 illustrates microscope images of picrosirius red-stained tissue sections showing collagen distribution changes in tissues after the treatment of inflammatory sites in mice with DNFB, dexamethasone, AAMM, AAMM-fraction 1, AAMM-fraction 2, AAMM-fraction 3, and AAMM-fraction 4 (x100 magnification).

As shown in FIG. 9, collapsed distribution of collagen was observed in the DNFB-induced inflammation group (0.2 % DNFB), while dense collagen distribution was found in the dexamethasone (0.2 %) treatment group and the AAMM (0.2 %) treatment group. Dense collagen distribution was also found in the AAMM-fraction 1 treatment group and the AAMM-fraction 4 treatment group, while less dense collagen distribution on the epidermal layer and distribution of irregular spaces were observed in the AAMM-fraction 2 and AAMM-fraction 3 treatment groups, showing slight breakdown of the regular distribution of collagen.

Table 3 comparatively shows physiological activities of *Artemisia annua* ethanol extract (AAMM) prepared in Example 1 and each AAMM-fraction prepared in Example 2 evaluated by tissue staining per category. The results of the microscopic histological observation were scored to comparatively represent degrees of efficacy for each category.

**[Table 3]**

| Category | Control group | DNFB | DM | AAMM | fraction 1 | fraction 2 | fraction 3 | fraction 4 |
|---|---|---|---|---|---|---|---|---|
| Hyperkeratosis | 0 | 3 | 1 | 1 | 1 | 2 | 1 | 1 |
| Acanthosis | 0 | 3 | 1 | 0 | 2 | 3 | 1 | 1 |
| Number of inflammatory cells | 0 | 3 | 0 | 0 | 0 | 3 | 1 | 1 |
| Collagen breakdown | 0 | 3 | 0 | 0 | 0 | 3 | 2 | 0 |

Score: None=0, Mild=1, Moderate=2, Severe=3
Control: DNFB-untreated, non-inflammatory group
DNFB: 0.2 % 1-Fluoro-2,4-dinitrobenzene (inflammation-inducing agent)
DM: 0.2 % Dexamethaxone (positive control group)
AAMM: Ethanol extract (0.2 %) of the residue obtained after extraction of *Artemisia annua* with dichloromethane in Example 1
fraction1: AAMM-fraction 1, a highly polar fraction (0.2 %) prepared with a 20 %-ethanol fractionating solvent in Example 2
fraction 2: AAMM-fraction 2, a medium-polar fraction (0.2 %) prepared with a 40 %-ethanol fractionating solvent in Example 2
fraction 3: AAMM-fraction 3, a medium-polar fraction (0.2 %) prepared with a 60 %-ethanol fractionating solvent in Example 2
fraction 4: AAMM-fraction 4, a low-polar fraction (0.2 %) prepared with a 100 %-ethanol fractionating solvent in Example 2

Through the above results, AAMM was found to exhibit a similar level of inflammation treatment efficacy as to dexamethasone known as a steroid dermatitis medicine at an equal dose. AAMM-fraction 1 and AAMM-fraction 4 prepared from the AAMM extract also exhibited significant dermatitis therapeutic efficacies, though slightly weaker than the efficacy of dexamethasone or AAMM at the same dose as dexamethasone.

### Test Example 3: Dermatitis relieving effects of AA extract prepared in Example 1 and AAMM-fraction 1+4 fraction prepared in Example 2

In order to investigate reasons for the slightly weaker efficacy of AAMM-fraction 1 and AAMM-fraction 4 than the AAMM parent extract, AAMM-fraction 1 and AAMM-fraction 4 were mixed in a weight ratio of about 9:1 in accordance with the yields thereof obtained from the AAMM extract, to thereby prepare "AAMM-fraction 1+4." Efficacy of AAMM-fraction 1+4 was compared with that of dexamethasone and AAMM. In order to further verify the significance of AAMM extract preparation, efficacy of *Artemisia annua* (AA) extracts prepared to contain most the components in AAMM extract and AAM extract prepared in Section 3 of Example 1 was compared with the efficacy of AAMM extract. The concentration of all the drugs was 0.2 %. Animal model preparation and drug treatment were carried out in the same manner as in Test Example 1. At 2 weeks after drug treatment, FDG uptake region of interest (ROI) values were calculated using an IVIS^{®} Spectrum animal imaging system with ¹⁸F-FDG tracer (Table 4) to compare efficacy of each sample. To obtain PET-CT images, all the mice were fasted with access only to water for about 12 hours. ¹⁸F-FDG of 37 MBq (1.0 mCi) was administered via the tail vein of the mice, and after 60 minutes, each mouse was subjected to image scanning for about 30 minutes.

Table 4 shows the FDG update ROI values, obtained using an IVIS^{®} Spectrum animal imaging system, of an *Artemisia annua* ethanol extract (AA) of Example 3, a mixture of AAMM-fraction 1 and AAMM-fraction 4 prepared in Example 2 in a ratio of about 9:1 (fraction 1+4), and other related test samples for dermatitis relieving efficiency comparison (at 0.2 % drug concentration in each sample) (units: ROI value, × 10⁵)

**[Table 4]**

| Samples | Control group | DNFB | DM | AA | AAMM | fraction 1 +4 |
|---|---|---|---|---|---|---|
| ROI after 2 weeks from drug administration | 6 | 25 | 11 | 22 | 13 | 12 |

Control group: DNFB-untreated, non-inflammatory group
DNFB: 0.2 % 1-Fluro-2,4-dinitrobenzene (inflammation-inducing agent)
DM: 0.2 % Dexamethaxone (positive control group)
AA: Ethanol extract (0.2 %) of dried *Artemisia annua* prepared in Example 3
AAMM: Ethanol extract (0.2 %) of the residue obtained after extraction of *Artemisia annua* with dichloromethane in Example 1
fraction1+4: 9:1 mixture (0.2 %) of AAMM-fraction 1 (highly polar fraction prepared with a 20 %-ethanol fractionating solvent) and AAMM-fraction 4 (low-polar fraction) prepared in Example 2

As shown in Table 4, a value of ROI indicative of the intercellular accumulation level of FDG in inflammation-induced regions was significantly lower in the dexamethasone treatment group and the AAMM treatment group than in the DNFB-induced inflammation group. Meanwhile, the AA extract was found to have weak efficacy. These results confirm that the preparation method of the AAMM extract, according to one or more embodiments, is a significant factor affecting the ameliorating effect of *Artemisia annua* on skin disorder such as dermatitis. The AAMM fraction 1+4 was found to exhibit a similar efficacy as to the AAMM extract, confirming that mixed use of AAMM fraction 1 and AAMM fraction 4 exhibits efficacy as good as the parent AAMM extract. Therefore, the composition for prevention, amelioration or treatment of a skin disorder, according to one or more embodiments, is considered to exhibit good efficacy equivalent to dexamethasone, due to the action of compounds or fractions having different functional mechanisms in combination, not a single mechanism of a single compound or a single fraction.

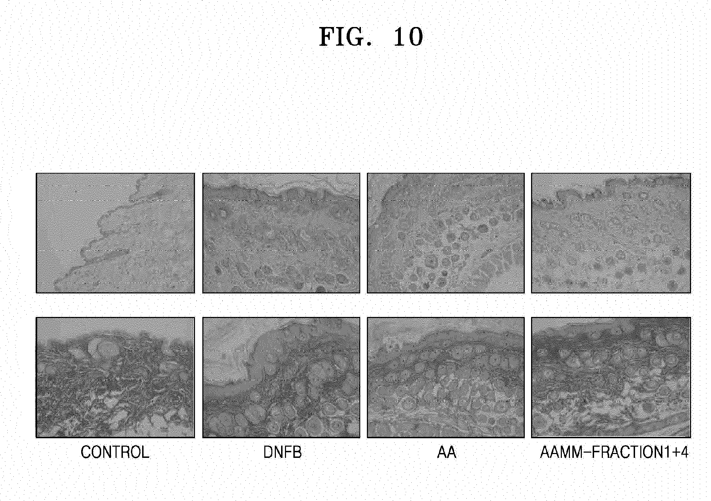

To test reliability of the values of ROI in the control group, the DNFB-induced inflammation group, the AA extract treatment group, and the AAMM-fraction 1+4 treatment group, skin cells of the mice of each group were taken and fixed with paraformaldehyde (pH 7.4) in the same manner as in Example 1. Then, the tissue samples were sectioned to a thickness of about 5 um, stained with hematoxylin and eosin (H&E), and then observed using optical microscopy (upper images in FIG. 10). The level of expression and distribution of collagen were microscopically observed by picrosirius red (PSR) staining of collagen in the tissue sections of inflammation-induced and drug treatment sites red (lower images in FIG. 10).

Referring to the upper images in FIG. 10, as a result of the microscopic observation of the inflammatory tissue sections after H&E staining, in the DNFB-induced inflammation group (0.2 % DNFB) and the AA extract treatment group, as compared with normal tissues (control group), a relatively thick keratin layer (darker color than the outermost boundary of the epidermis) on the epidermal surface, and acanthosis and inflammatory cells in the epidermal layer were observed. Meanwhile, in the AAMM-fraction 1+4 treatment group, thin distribution of keratin on the epidermal surface, nearly no acanthosis and a very small number of inflammatory cells were observed.

Referring to the lower images in FIG. 10, as a result of straining inflammatory tissues red to observe collagen distribution in the inflammatory tissues, in the DNFB-induced inflammation group (0.2 % DNFB) and the AA extract treatment group, collapsed distribution of collagen under the surface layer or greatly reduced density of collagen in the epidermal layer was observed. Meanwhile, the AAMM-fraction 1+4 treatment was found to have a dense distribution of collagen in the epidermal layer.

### Test Example 4: In vitro autophagy induction activity of single-compound mixtures prepared in Example 4

In order to confirm reasons for the slightly weaker efficacy of fraction 1 and fraction 4 than the AAMM patent extract as found in Test Example 1 and to test the effectiveness of using a composite composition of single compounds according to embodiments, not single compounds alone, physiological activities of single compounds of Example 3 in human keratinocyte cell line, HaCaT cells, and *in vitro* autophagy induction activities of mixtures (Example 4) with different compositions of the single compounds of Example 3 were compared.

HaCaT cells were cultured in a Dulbecco's Modified Eagle's medium (DMEM, Hyclone, Utah, US) supplemented with 10 % fetal bovine serum (FBS) and 0.1 % of penicillin (Streptomycin) and used in experiments. The cells were adapted and incubated in a humidified incubator of about 5 % carbon dioxide at about 37 °C.

Prior to autophagy induction activity measurement, cytotoxicity assessment of cells in test samples was performed by methyl thiazolyl tetrazolium (MTT) assay. HaCaT cells were appropriately dispersed onto a 96-well plate at a concentration of 4×10⁴ cells/mL and cultured for about 24 hours for fixation and stabilization. After the 24-hour incubation was complete, a culture broth was diluted until a final concentration of each compound or each mixture reached about 10 µg/mL, about 50 µg/mL, and 100 µg/mL, supplied onto the fixed and stabilized cell line, and then incubated for about 24 hours. After the 24-hour incubation, the culture medium was removed, and a MTT solution (0.5 mg/mL in PBS) was added by about 100 mL each time, followed by further incubation for reaction in a humidified incubator of about 5 % carbon dioxide at about 37 °C for about 30 minutes until MMT was reduced. Formazan crystals generated in each well were dissolved with 200 mL of dimethylsulfoxide (DMSO), and absorbance at 570 nm was measured using a microplate reader (Bio-Rad, USA) to thereby measure cell viability relative to the non-drug treatment group (Negative Control).

As a result of the MTT cytotoxicity assay of the test samples on HaCaT cell lines, all the single compounds and mixtures were found to exhibit a cell viability of about 80 % or greater, indicating that all the test samples have no notable cytotoxicity.

To measure autophagy induction activity, HaCaT cells were dispersed onto a 60-mm Petri dish and incubated. After the 24-hour incubation, rapamycin, a comparative drug, was diluted with the culture broth until a final concentration of about 250 nM was reached, and then each compound or each mixture was diluted with the culture broth until a final concentration of about 1 µg/mL or about 10 µg/mL was reached. Then, the resulting culture broth was supplied onto the fixed and stabilized cell lines and incubated for about 12 hours. Then, the incubated cells were recovered, dissolved in a radio-immunoprecipitation assay (RIPA) lysis buffer, and then centrifuged at about 12,000 rpm for about 30 minutes to thereby isolate proteins. The isolated proteins were quantified using Bradford method (Bio-Rad Laboratories, CA, USA). The quantified proteins were subjected to electrophoresis on a 12 % polyacrylamide gel by sodium dodecyl sulfate polyacrylamide gel electrophoresis (SDS-PAGE), and then transferred onto a nitrocellulose membrane. The nitrocellulose membrane was then reacted with a primary antibody anti-LC3 at about 4 °C for about 1 day, washed with TBS-T three times for about 10 minutes, and then reacted with a horseradish peroxidase-linked (HRP) secondary antibody for about 1 hour. After reaction with the antibodies was complete, the reaction product was washed with TBS-T solution and reacted with enhanced chemiluminescence (ECL) Western Blot reagents (GE Healthcare, Chalfont St. giles UK), followed by detection with a Chemidoc system (Bio-rad, USA). The degree of expression of autophagy-measuring marker LC3B and an expression ratio of LC3B to LC3A in each test sample were comparatively analyzed.

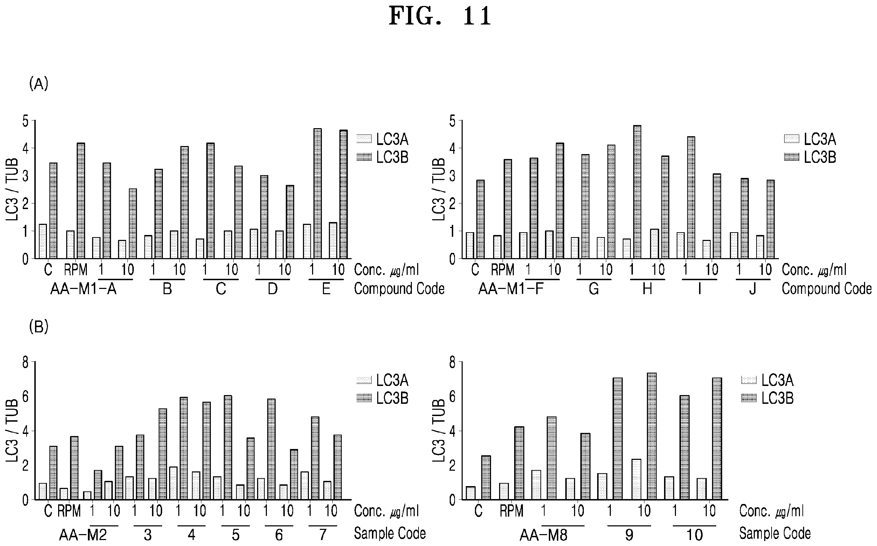

As shown in FIG. 11, each single compound according to one or more embodiments was found to exhibit an increase in LC3B expression (LC3B as an autophagy activity indicator) at a level equal to or below that of rapamycin (a concentration of about 250 nM), an autophagy drug used as a positive control group. Meanwhile, the AA-M4 composition (at concentrations of 1 µg/mL and 10 µg/mL) as a mixture of unsaturated fatty acids, including α-linolenic acid, and hexadecanoic acid as saturated fatty acid; the AA-M5 composition (at a concentration of 1 µg/mL) as a mixture of chlorogenic acid and unsaturated fatty acids; the AA-M6 composition (at a concentration of 1 µg/mL) as a mixture of ornithine, pinitol, chiro-inositol, and betaine; and the AA-M9 composition and the AA-M10 composition (at concentrations of 1 µg/mL and 10 µg/mL), each as a mixture of seven single compounds, exhibited a significant autophagy protective activity in skin cells of about 1.5 times or greater than the activity of rapamycin. The AA-M9 composition and the AA-M10 composition of the physiologically active samples exhibited a comparatively high degree of LC3B expression. In view of the expression ratio of LC3B to LC3A, the AA-M9 composition (at a concentration of 1 µg/mL) and the AA-M10 composition (at concentrations of 1 µg/mL and 10 µg/mL) were found to induce the greatest autophagosome formation.

### Test Example 5: Dermatitis relieving effects of single-compound mixtures prepared in Example 4

To test the *in vivo* efficacy of ameliorating dermatitis with the compositions which exhibited a comparatively high *in vitro* autophagy induction activity in Test Example 4, efficacies of the AA-M6 composition and the AA-M9 composition among the physiologically active samples used in Test Example 4 were evaluated at a concentration of 0.3 % in DMSO in the DNFB-induced dermatitis mouse models. The AA-M7 composition (0.3 % in DMSO), which exhibited a relatively weak physiological activity than other mixtures in Test Example 4, was used as a comparative group, and dexamethasone (0.15 % in DMSO) was used as a positive control group. Efficacies of a control group treated with neither a solvent nor a drug, and a solvent control group treated with DNFB and then only DMSO solvent were also measured and compared with the test groups.

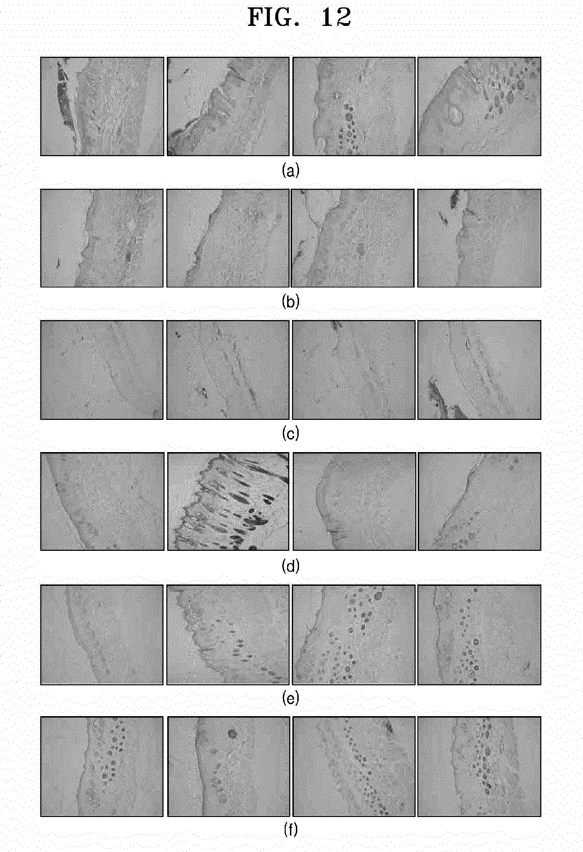

To measure efficacies of the samples, NC/Nga atopic dermatitis mouse models (6-week aged males having a body weight of about 30 g per subject, available from Central Laboratory Animal Inc.), housed in an air flow-through sterile test bench, and freely fed with standard solid feeds for 1 week. For use in experiments, all test reagents were dissolved in a small amount of DMSO until a final concentration of 0.3 % was reached. Dexamethasone was dissolved in DMSO at a concentration of about 0.15 % and used as a positive control group. Each of the control groups and test groups consisted of four mice. To induce skin itching and atopic dermatitis, on the 1^{st} day immediately after the back hair of each mouse was removed, 0.15 % of DNFB dissolved in a mixed solvent of acetone and olive oil (3:1 by volume) was applied onto the hair-removed back. Then, 0.15 % of DNFB was applied again onto the back on the 5^{th} day, and 0.2 % of DNFB was applied thereonto on the 9^{th} day, followed by treatment with test drugs. Then, each mouse was treated with 0.2 % of DNFB and then each test drug every two days till the 30^{th} day. Next, skin cells of the mice were taken and fixed with paraformaldehyde (pH 7.4) in the same manner as in Example 1. Then, the tissue samples were sectioned to a thickness of about 5 um, stained with hematoxylin and eosin (H&E), and then observed using optical microscopy (FIG. 12). FIG. 12 illustrates the results of microscopic observation after hematoxylin and eosin (H&E) staining of skin tissue sections in which DNFB-induced inflammatory sites were treated with the AA-M6 composition and the AA-M9 composition.

In FIG. 12, (a), (b), (c), (d), (e), and (f) are histopathological microscope images by hematoxylin and eosin (H&E) of a mouse group treated with 0.2% DNFB, a mouse group treated with 0.2% of DNFB and then only DMSO solvent, a mouse group treated with 0.2% of DNFB and then 0.15% of dexamethasone as a positive control group, a mouse group treated with 0.2% of DNFB and then 0.3% of the AA-M6 composition, a mouse group treated with 0.2% of DNFB and then 0.3% of the AA-M7 composition, and a mouse group treated with 0.2% of DNFB and then 0.3% of the AA-M9 composition, respectively, wherein each mouse group consisted of four mice.

As shown in (a) and (b) in FIG. 12, in the DNFB-induced inflammation group (0.2 % DNFB) and the DNFB and DMSO treatment group, as compared with normal tissues (control group), a relatively thick keratin layer on the epidermal surface, and acanthosis and inflammatory cells appearing as relatively dark blue lines or an aggregate of small dots in the epidermal layer were observed. Referring to (c) in FIG. 12, in the dexamethasone treatment group as a positive control group, thin distribution of keratin on the epidermal surface, nearly no acanthosis, and a very small number of inflammatory cells were observed, and thus tissue morphology in which inflammation and itching were significantly relieved was observed. Referring to (d) in FIG. 12 (AA-M6 composition treatment group) and (e) in FIG. 12 (AA-M7 composition treatment group), a relative thick keratin layer as compared with the dexamethasone treatment group, and signs of acanthosis under the keratin layer were observed indicating a weak efficacy. Meanwhile, as shown in (f) in FIG. 12, in the AA-M9 composition treatment group, comparative thin distribution of keratin layer and less acanthosis were observed, indicating an improved efficacy of ameliorating inflammation, as compared with the AA-M6 composition treatment group and the AA-M7 composition treatment group.

### Test Example 6: Efficacy of ethyl acetate extract and ethanol extract prepared from residue remaining after extraction with ethyl acetate

According to the processes as described in Section 4 of Example 1 subtitled "4. Preparation of extracts with different solvents," an ethyl acetate extract (hereinafter, also referred to as 'AAE1') of *Artemisia annua* originating from Korea and a 90 % ethanol extract (hereinafter, also referred to as 'AAEM1') prepared from the residue remaining after extraction with ethyl acetate were obtained.

Next, HaCaT cells, a human keratinocyte cell line, were incubated in the presence of AAE1 and AAEM1, and level of interleukin-1β (IL-1β), an anti-inflammatory indication, was measured.

Cytotoxicity assessment of cells in test samples was performed by methyl thiazolyl tetrazolium (MTT) assay. HaCaT cells were appropriately dispersed onto a 96-well plate at a concentration of 3×10⁴ cells/mL and cultured for about 24 hours for fixation and stabilization. After the 24-hour incubation was complete, extracts were added thereto at a concentration of about 10 µg/mL, about 50 µg/mL, or 100 µg/mL, and then incubated in a CO₂ incubator for about 24 hours. After the 24-hour incubation, the culture medium was removed, and a MTT solution (0.5 mg/mL in PBS) was added by about 100 mL each time, followed by further incubation for reaction in a humidified incubator of about 5 % carbon dioxide at about 37 °C for about 1 hour until MMT was reduced. After removing the MMT reagents, the reduced MMT were dissolved with 200 mL of dimethylsulfoxide (DMSO), and absorbance at 570 nm was measured using a microplate reader (Bio-Rad, USA) to thereby measure cell viability relative to the non-drug treatment group (Negative Control). As a result, all the extracts were found to exhibit a cell viability of about 80 % or greater at the test concentration, indicating that all the test samples have no notable cytotoxicity.

For measurement of IL-1β secretion, HaCaT cells were dispersed onto a 48-well plate at a concentration of about 6×10⁴/mL, stabilized for about 24 hours, and then treated with each extract at different concentrations. After about 1 hour, the cells were activated with TNF-α (10 ng/mL) and IFN-γ (10 ng/mL) and incubated in a CO₂ incubator for about 24 hours. The resulting culture broth was subjected to enzyme-linked immunosorbent assay (ELISA) with an ELISA KIT according to the instructions of the manufacturer. Cytokine secretion was analyzed with a micro-spectrophotometer at a wavelength of 450 nm.

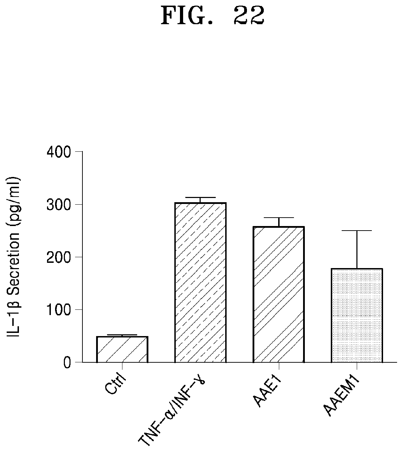

FIG. 22 is a graph illustrating the effect of AAE1 and AAEM1 on expression of IL-1β in cells. Referring to FIG. 22, AAEM1 was found to significantly reduce the expression of IL-1β, as compared with AAE1, and have anti-inflammatory effect. In FIG. 22, "Ctrl" denotes a negative control group in which only the medium was used, and "TNF-a/INF-r" denotes a positive control group in which TNF-α and INF-r were added to the medium at a concentration 10 ng/mL each.

HaCaT cells, a human keratinocyte cell line, were incubated in the presence of AAE1 or AAEM1 under autophagy induction conditions, and the level of LC3, an autophagy marker, was measured. The incubation of HaCaT cells was performed under the same conditions as applied in Test Example 4 for the autophagy assay, except that about 10 ug/mL of each extract was included in the medium. The level of LC3 was measured by western blotting.

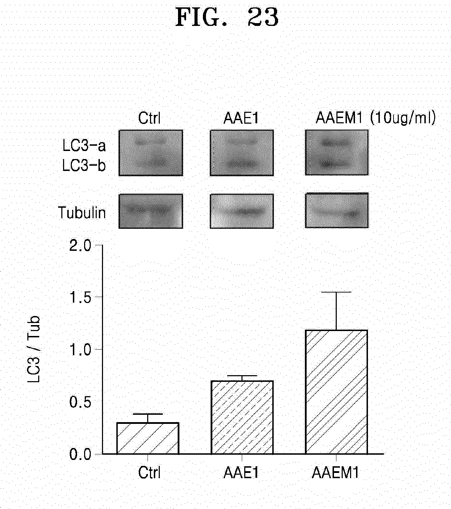

FIG. 23 illustrates the effect of AAE1 and AAEM1 on the expression of LC3 in the cells. Referring to FIG. 23, AAEM1 was found to significantly increase the expression of LC3, as compared with AAE1, and have cell protective action. In FIG. 23, "Ctrl" denotes a negative control group in which only the medium was used, and "Tub," which means tubulin, denotes a reference value for LC3 level measurement.

### MODE OF THE INVENTION

According to an aspect of the disclosure, a method of isolating an *Artemisia annua* extract, the method including contacting *Artemisia annua* with a low-polarity solvent to extract a component of the *Artemisia annua* in the low-polarity solvent, wherein the low polarity solvent is dichloromethane, diethyl ether, ethyl acetate or acetone; separating and removing the low-polarity solvent from the mixture to obtain a residue; and contacting the residue with a hydrophilic solvent to extract a component of the *Artemisia annua* in the hydrophilic solvent, wherein the hydrophilic solvent is a C1-C6 alcohol, water, or a combination thereof, and wherein the *Artemisia annua* extract comprises at least four compounds selected from the group consisting of ornithine, pinitol, muco-inositol, chiro-inositol, betaine, and chlorogenic acid.

The *Artemisia annua* may be used as a whole or a part thereof may be used. The part of the *Artemisia annua* may be aerial part or underground part. The aerial part may be leaves, flowers, fruits, or a combination thereof. The underground part may be underground stems, roots, or a combination thereof. The *Artemisia annua* may be in a dried state. The *Artemisia annua* may be commercially purchased, collected from nature, or cultivated for use.

The term "extract" used herein may mean a crude extract used in the art, or broadly a fraction obtained by additional fractionation of the extract. Accordingly, the extract may be a crude extract, a fraction, or a combination thereof. The crude extract may mean an extract obtained by contacting the *Artemisia annua* with an extraction solvent and not separated to include a specific component. The fraction may mean a portion including a specific component separated from the crude extract. The extract or a fraction thereof may include an *Artemisia annua* extract, a fraction thereof, or a subfraction thereof, which may be included alone or as a mixture thereof, or may include a separated compound alone or as a main ingredient. The subfraction may be obtained by using an ultrafiltration membrane having a cut-off value.

In one or more embodiments, the separating may be performed by filtration, immersion, centrifugation, chromatography, or a combination thereof. The chromatography, which is designed for separation according to various conditions, i.e., by size, charge, hydrophobicity, or affinity, may be ion exchange chromatography, affinity chromatography, size exclusion chromatography, high-performance liquid chromatography (HPLC), high speed fluid chromatography, column chromatography, reverse-phase column chromatography, or a combination thereof. The separating and removing of the low-polarity solvent may include separating a residue remaining after the extraction of the component of *Artemisia annua* in the mixture of the *Artemisia annua* and the low-polarity solvent by contact of the *Artemisia annua* and the low-polarity solvent, from the low-polarity solvent, and then removing the remaining low-polarity solvent.

The extraction may include incubation of the *Artemisia annua* in a solvent for a predetermined period of time. The extraction may be performed with or without stirring, or may include heating. The incubation may be performed at room temperature or a reflux temperature with or without stirring. The incubation temperature may be appropriately chosen according to a selected solvent. For example, the incubation temperature may be from room temperature to a reflux temperature, from 30 °C to a reflux temperature, or from 40 °C to a reflux temperature. The heating may include heating to about 50 °C, about 60 °C, about 70 °C, about 80 °C, or a reflux temperature. The heating may be heating to a temperature from about 50 °C to a reflux temperature, a temperature from about 60 °C to a reflux temperature, a temperature from about 70 °C to a reflux temperature, a temperature from about 80 °C to a reflux temperature, r a reflux temperature. The extraction time, which may be varied depending on a selected temperature, may be from about 1 hour to 2 months. For example, the extraction time may be from about 1 hour to 1 month, about 1 hour to 15 days, about 1 hour to 10 days, about 1 hour to 5 days, about 1 hour to 3 days, about 1 hour to 2 days, about 1 hour to 1 day, about 5 hours to 1 month, about 5 hours to 15 days, about 5 hours to 10 days, about 5 hours to 5 days, about 5 hours to 3 days, about 5 hours to 2 days, about 5 hours to 1 day, about 10 hours to 1 month, about 10 hours to 15 days, about 10 hours to 10 days, about 10 hours to 5 days, about 10 hours to 3 days, or about 10 hours to 2 days. The extraction may be reflux extraction of the *Artemisia annua* in a solvent. A volume of the solvent may be about at least 1 time, at least 2 times, at least 5 times, at least 10 times, or at least 15 times a weight of the *Artemisia annua.* For example, the volume of the solvent may be about 1 time to 15 times, about 2 times to about 15 times, about 5 times to about 15 times, about 10 times to about 15 times, or about 15 times the weight of the *Artemisia annua.* The *Artemisia annua* may be dried in the shade.

The extraction may be performed using a common method used in the art, for example, filtration, hot-water extraction, immersion extraction, cold water extraction, microwave extraction, reflux cooling extraction, pressure extraction, subcritical extraction, supercritical extraction, or ultrasonic extraction. For example, the extraction may be performed using immersion extraction. The immersion extraction may be warm or heated immersion or room-temperature immersion, and may include 1 time to 5 times of extraction. The *Artemisia annua* may be contacted with an extraction solvent in a volume of about 0.1 time to 10 times or about 1 time to 6 times of weight of the *Artemisia annua.* The temperature of the cold water immersion extraction may be about 20 °C to about 40 °C. The temperature of the warm or heated immersion extraction may be about 40 °C to about 100 °C. The cold water immersion extraction time may be about 24 hours to about 120 hours. The warm or heated immersion extraction time may be about 0.5 hour to about 48 hours.

The extraction may include removing a solvent from an extract solution by using a known method such as reduced pressure concentration. The extraction may include drying the obtained extract, for example, by freeze drying to thereby prepare a dry extraction. The reduced pressure concentration may be performed using a vacuum reduced pressure concentrator or a vacuum rotary evaporator. The drying may be reduce pressure drying, vacuum drying, boil drying, spray drying, or freeze drying.

The composition for use including the *Artemisia annua* extract according to a preferred embodiment may include a low content of sesquiterpene lactone or may nearly not include sesquiterpene lactone. The low-polarity solvent may dissolve or extract the sesquiterpene lactone, so that the sesquiterpene lactone may be removed from the *Artemisia annua* extract by the removal of the low-polarity solvent from the mixture of the *Artemisia annua* and the low-polarity solvent after the extraction of a component of the *Artemisia annua.* The sesquiterpene lactone may have a structure containing 15 carbon atoms and a lactone ring, wherein the number of carbon atoms may vary according to derivatives thereof or subcaterories of the sesquiterpene lactone. The sesquiterpene lactone may include germacranolides, heliangolides, guaianolides, pseudoguaianolides, hypocretenolides, or eudesmanolides.

The sesquiterpene lactone is relatively soluble and is eluted in the low-polarity solvent or non-polar solvent.

The composition for use including the *Artemisia annua* extract may include a low content of an essential oil component or may nearly not include an essential oil component, as in the case of the sesquiterpene lactone. Accordingly, the composition for use including the *Artemisia annua* extract may include a low content of sesquiterpene lactone, an essential oil component, and a combination thereof or may nearly not include the same.

In the method of isolating the *Artemisia annua* extract, the hydrophilic solvent is capable of dissolving and extracting at least four compounds selected from the group consisting of ornithine, pinitol, muco-inositol, chiro-inositol, betaine and chlorogenic acid. The hydrophilic solvent may also be capable of dissolving and extracting at least one selected from the group consisting of α-linolenic acid, linoleic acid, and palmitic acid.

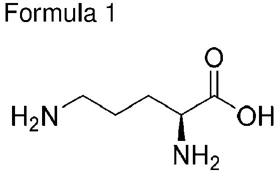

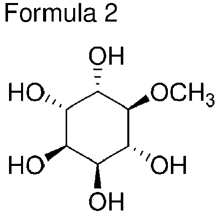

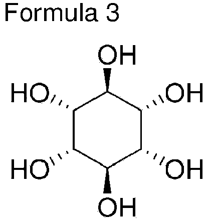

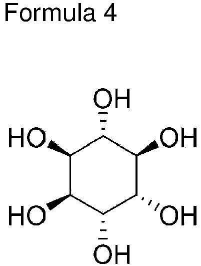

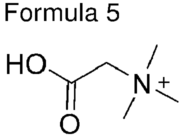

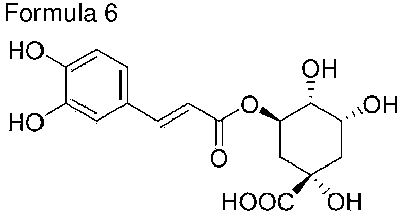

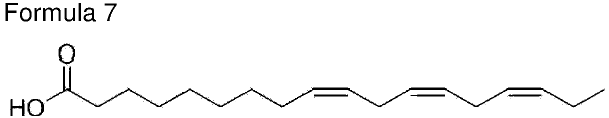

The compound of Formula 1 may be named ornithine, the compound of Formula 2 may be named pinitol, The compound of Formula 3 may be named muco-inositol, the compound of Formula 4 may be named chiro-inositol, the compound of Formula 5 may be named betaine, the compound of Formula 6 may be named chlorogenic acid, and the compound of Formula 7 may be named α-linolenic acid.

The hydrophilic solvent is a C1-C6 alcohol, water, or a combination thereof. The alcohol may be a lower alcohol, for example, a C1-C3, C1-C4, C1-C5, or C1-C6 compound having at least one -OH group. For example, the C1-C4 alcohol may be methanol, ethanol, propanol, isopropanol, butanol, sec-butanol, isobutanol, tert-butanol, or a combination thereof.

In one or more embodiments, the method may further include contacting the *Artemisia annua* extract with a fractionating solvent to fractionate the *Artemisia annua* extract. In one or more embodiments, the fractionating may include contacting the *Artemisia annua* extract with a chromatography medium to be bound thereto and eluted *annua* with a fractionating solvent.

The fractionating solvent may be a hydrophilic solvent. In one or more embodiments, the hydrophilic solvent may be a lower alcohol or a combination of lower alcohols. The lower alcohol may be a C1-C3, C1-C4, C1-C5, or C1-C6 compound having at least one -OH group. In one or more embodiments, the C1-C4 alcohol may be methanol, ethanol, propanol, isopropanol, butanol, sec-butanol, isobutanol, tert-butanol, or a combination thereof.

The fractionating solvent may contain about 0 vol% to 100 vol% of alcohol with respect to a total volume of the fractionating solvent. Accordingly, a volume ratio of alcohol to water may be about 0:10, about 1:9, about 2:8, about 3:7, about 4:6, about 5:5, about 6:4, about 7:3, about 8:2, about 9:1, or about 10:0. In the volume ratios, a ratio of '0' means pure water or pure alcohol. For example, when a volume ratio of alcohol to water is 2:8, it may mean about 20 vol% of alcohol. In one or more embodiments, the fractionating may include eluting with a fractionating solvent containing about 20 vol% or greater to less than 40 vol%, about 40 vol% or greater to less than 60 vol%, about 60 vol% or greater to less than 100 vol%, or about 100 vol% of alcohol with respect to a total volume of the fractionating solvent. Accordingly, fractions resulting from the fractionation may be fractions obtained with fractionating solvents containing about 20vol%, 40vol%, 60vol%, or 100vol% of alcohol, respectively, with respect to a total volume of the fractionating solvent. In one or more embodiments, the alcohol may be a lower alcohol. The lower alcohol may be a C1-C3, C1-C4, C1-C5, or C1-C6 compound having at least one -OH group. In one or more embodiments, the lower alcohol may be ethanol.

According to another aspect of the disclosure, a composition for use in the prevention, amelioration, or treatment of a skin disorder includes the *Artemisia annua* extract prepared by the *Artemisia* extract isolation method according to any of the embodiments, wherein the skin disorder is at least one inflammatory skin disorder selected from the group consisting of skin pruritus, seborrheic dermatitis, contact dermatitis, deciduous dermatitis, hidradenitis, atopic dermatitis, drug allergy, diabetic dermatitis, bacterial dermatitis, fungal dermatitis, allergic dermatitis and neurogenic dermatitis, and wherein the *Artemisia annua* extract comprises at least four compounds selected from the group consisting of ornithine, pinitol, muco-inositol, chiro-inositol, betaine, and chlorogenic acid.

Since the method of isolating the *Artemisia annua* extract, according to any of the embodiments, includes extracting sesquiterpene lactone with a low-polar or non-polar solvent capable of dissolving the sesquiterpene lactone and isolating and removing the sesquiterpene lactone, the *Artemisia annua* extract according to any of the embodiments, a fraction thereof, or a subfraction thereof may include a low content of sesquiterpene lactone or may substantially not include sesquiterpene lactone. Since the low-polar or non-polar solvent used in the method according to any of the embodiments is capable of dissolving an essential oil component, like the sesquiterpene lactone, an essential oil component may be extracted, separated, and removed from the *Artemisia annua* extract according to any of the embodiments. Accordingly, the *Artemisia annua* extract according to any of the embodiments, a fraction thereof, or a subfraction thereof may include a low content of sesquiterpene lactone, an essential oil component, and a combination thereof, or may substantially not include sesquiterpene lactone, an essential oil component, and a combination thereof. In one or more embodiments, the composition may include sesquiterpene lactone in an amount of about 1.00 % to about 0.001 %, about 0.80% to about 0.001 %, about 0.60 % to about 0.001 %, about 0.50 % to about 0.001 %, about 0.40 % to about 0.001 %, about 0.30 % to about 0.001 %, about 0.20 % to about 0.001 %, about 0.10 % to about 0.001 %, about 0.08 % to about 0.001 %, about 0.06 % to about 0.001 %, about 0.05 % to about 0.001 %, about 0.04 % to about 0.001 %, about 0.03 % to about 0.001 %, about 0.02 % to about 0.001 %, about 0.01 % to about 0.001 %, about 0.1 % or less, 0.05 % or less, 0.01 % or less, 0.005 % or less, or about 0.001 % or less by weight (w/w). In one or more embodiments, the composition may include an essential oil component in an amount of about 1.00% to about 0.01 %, about 0.80% to about 0.01 %, about 0.60% to about 0.01 %, about 0.50 % to about 0.01 %, about 0.40 % to about 0.01 %, about 0.30 % to about 0.01 %, about 0.20 % to about 0.01 %, about 0.10 % to about 0.01 %, about 0.08 % to about 0.01 %, about 0.06 % to about 0.01 %, about 0.05 % to about 0.01 %, about 0.04 % to about 0.01 %, about 0.03 % to about 0.01 %, about 0.02 % to about 0.01 %, about 0.1 % or less, about 0.05 % or less, about 0.01 % or less, about 0.005 % or less, or about 0.001 % or less by weight (w/w). The essential oil component may be a volatile essential oil component.

In one or more embodiments, the *Artemisia annua* extract may further include at least one compound selected from the group consisting of linoleic acid, palmitic acid, and α-linolenic acid.

These combinations of the compounds may be included in the fractions obtained by fractionation with fractionating solvents containing about 20vol% or greater to less than 40 vol%, about 40 vol% or greater to less than 60vol%, about 60 vol% or greater to less than 100 vol%, and about 100 vol% of alcohol, respectively, with respect to a total volume of the fractionating solvent. The fractions obtained by fractionation with the fractionating solvents containing about 20vol% or greater to less than 40vol%, about 40 vol% or greater to less than 60vol%, about 60 vol% or greater to less than 100 vol%, and about 100 vol% of alcohol, respectively, with respect to a total volume of the fractionating solvent may include ornithine, pinitol, muco-inositol, chiro-inositol, betaine, chlorogenic acid, or a combination thereof as a major component. The fractions obtained by fractionation with a fractionating solvent containing 100 vol% of alcohol may include linoleic acid, palmitic acid, α-linolenic acid, or a combination thereof as a major component. Since the content of each compound in each fraction may vary, the fractions may be stirred or mixed at an appropriate ratio to achieve optimal pharmacological effects. The composition and ratio of fractions may be varied as needed. For example, assuming that the fractions obtained by fractionation with the fractionating solvents contain about 20 vol% or greater to less than 40 vol%, about 40 vol% or greater to less than 60 vol%, about 60 vol% or greater to less than 100 vol%, and about 100 vol% of alcohol, respectively, and are named a first fraction, a second fraction, a third fraction, and a fourth fraction, respectively, the composition may be a mixture of the first fraction and the second fraction, a mixture of the first fraction and the third fraction, a mixture of the first fraction and the fourth fraction, a mixture of the second fraction and the third fraction, a mixture of the second fraction and the fourth fraction, or a mixture of the third fraction and the fourth fraction; a mixture of the first fraction, the second fraction and the third fraction, a mixture of the first fraction, the third fraction and the fourth fraction, or a mixture of the second fraction, the third fraction and the fourth fraction; or a mixture of the first fraction, the second fraction, the third fraction, and the fourth fraction. In the mixtures of two of the fractions, a weight ratio of the two fractions may be about 1:9, about 2:8, about 3:7, about 4:6, about 5:5, about 6:4, about 7:3, about 8:2, or about 1:9. In the mixtures of three of the fractions, a weight ratio of the three fractions may be about 1:1:8, about 1:2:7, about 1:3:6, about 1:4:5, about 1:5:4, about 1:6:3, about 1:7:2, about 1:8:1, about 2:1:7, about 2:2:6, about 2:3:5, about 2:4:4, about 2:5:3, about 2:6:1, about 2:7:1, about 3:1:6, about 3:2:5, about 3:3:4, about 3:4:3, about 3:5:2, about 3:6:1, about 4:1:5, about 4:2:4, about 4:3:3, about 4:4:2, about 4:5:1, about 5:1:4, about 5:2:3, about 5:3:2, about 5:4:1, about 6:1:3, about 6:2:2, about 6:3:1, about 7:1:2, about 7:2:1, or about 8:1:1. In the mixtures of four of the fractions, a weight ratio of the four fractions may be about 1:1:1:7, about 1:2:1:6, about 1:3:1:5, about 1:4:1:4, about 1:5:1:3, about 1:6:1:2, about 1:7:1:1, about 1:1:2:6, about 1:1:3:5, about 1:1:4:4, about 1:1:5:3, about 1:1:6:2, about 1:1:7:1, about 2:1:1:6, about 2:2:1:5, about 2:3:1:4, about 2:4:1:3, about 2:5:1:2, about 2:6:1:1, about 2:1:2:5, about 2:1:3:4, about 2:1:4:3, about 2:1:5:2, about 2:1:6:1, about 3:1:1:5, about 3:2:1:4, about 3:3:1:3, about 3:4:1:2, about 3:5:1:1, about 3:1:2:4, about 3:1:3:3, about 3:1:4:2, about 3:1:5:1, about 4:1:1:4, about 4:2:1:3, about 4:3:1:2, about 4:4:1:1, about 4:1:2:3, about 4:1:3:2, about 4:1:4:1, about 5:1:1:3, about 5:2:1:2, about 5:3:1:1, about 5:1:2:2, about 5:1:3:1, about 6:1:1:2, about 6:2:1:1, about 6:1:2:1, or about 7:1:1:1.

In one or more embodiments, when the composition for use includes a combination of at least four compounds selected from the group consisting of ornithine, pinitol, muco-inositol, chiro-inositol, betaine, and chlorogenic acid; and at least one compound selected from the group consisting of linoleic acid, palmitic acid, and α-linolenic acid, the composition may be a mixture or combination of a fraction obtained by fractionation with a fractionating solvent containing about 20 vol% or greater to less than 40 vol% of alcohol with respect to a total volume of the fractionating solvent, and a fraction obtained by fractionation with a fractionating solvent containing 100 vol% of alcohol. Accordingly, the composition for use may include either one of the above-described fractions alone or may include a combination of two of the fractions in a weight ratio of about 1:9, about 2:8, about 3:7, about 4:6, about 5:5, about 6:4, about 7:3, about 8:2, or about 1:9. In one or more embodiments, the composition may include a combination of two of the fractions in a weight ratio of about 9:1.

The *Artemisia annua* extract, a fraction thereof, or a compound isolated therefrom inhibits inflammatory reactions. Accordingly, in the composition for use, the *Artemisia annua* extract is included in an effective amount for the inhibition of inflammatory reactions which cause skin disorders or for the recovery of wounds. The effective amount may be, per 1,000 mg of the composition, about 0.1 mg to about 1,000 mg, for example, about 0.1 mg to about 500 mg, about 0.1 mg to about 100 mg, about 0.1 mg to about 50 mg, about 0.1 mg to about 25 mg, about 1 mg to about 1,000 mg, about 1 mg to about 500 mg, about 1 mg to about 100 mg, about 1 mg to about 50 mg, about 1 mg to about 25 mg, about 5 mg to about 1,000 mg, about 5 mg to about 500 mg, about 5 mg to about 100 mg, about 5 mg to about 50 mg, about 5 mg to about 25 mg, about 10 mg to about 1,000 mg, about 10 mg to about 500 mg, about 10 mg to about 100 mg, about 10 mg to 50 mg, or about 10 mg to about 25 mg. The composition may be for the inhibition of inflammatory reactions *in vitro* or in cells of a subject. The cells of a subject may be, for example, cells at a site in which an inflammatory reaction is in progress or is expected to occur, or cells at a site in which incidence of a disease caused by an inflammatory reaction is apparently observed.

In one or more embodiments, the skin disorder may be dermatitis. Examples of the skin disorder may include diseases such as skin itching, urticaria, atopy, dryness dermatitis, neurogenic dermatitis, and contact dermatitis. The skin itching may indicate unbearable scratching caused by insect bites or certain contact to which the skin is sensitive or caused by the skin nerves being weakly and continuously irritated due to atopy, dermatitis, psoriasis, infections, skin dryness, or skin aging. The urticaria may indicate a skin rash caused by severe mental stress, foods or drugs, or allergic inflammation caused by allergens. The atopy may indicate allergic inflammatory diseases with rash, eczema, and severe itching caused by overly sensitive immune reactions of the skin even without direct contact to allergens. The dryness dermatitis may indicate dermatitis symptoms that have been exacerbated by dryness eczema or senile eczema due to increased skin dryness, with much shedding of skin. The neurogenic dermatitis may indicate that nerves become overly sensitive such that even minor irritation leads to severe itching, resulting in thickening of the stratum corneum and skin wrinkles, which consequently worsens itching and leads to continuous scratching that aggravates symptoms of dermatitis such as eczema. The contact dermatitis may mean symptoms with a local rash or irritation and itching when exposed to allergens or certain stimuli.

The composition for use includes the *Artemisia annua* extract and may include a compound isolated therefrom by an additional process. The compound may be in the form of a stereoisomer thereof, a solvate thereof, or a pharmaceutically acceptable salt thereof. The composition for use may include five compounds, or six compounds. The four compounds may be ornithine, pinitol, muco-inositol, and chiro-inositol; ornithine, pinitol, muco-inositol, and betaine; ornithine, pinitol, muco-inositol, and chlorogenic acid; ornithine, pinitol, chiro-inositol, and betaine; ornithine, pinitol, chiro-inositol, and chlorogenic acid; ornithine, pinitol, betaine, and chlorogenic acid; ornithine, muco-inositol, chiro-inositol, and betaine; ornithine, muco-inositol, chiro-inositol, and chlorogenic acid; ornithine, muco-inositol, betaine, and chlorogenic acid; ornithine, chiro-inositol, betaine, and chlorogenic acid; pinitol, muco-inositol, chiro-inositol, and betaine; pinitol, muco-inositol, chiro-inositol, and chlorogenic acid; pinitol, muco-inositol, betaine, and chlorogenic acid; pinitol, chiro-inositol, betaine, and chlorogenic acid; or muco-inositol, chiro-inositol, betaine, and chlorogenic acid. The five compounds may be ornithine, pinitol, muco-inositol, chiro-inositol, and betaine; ornithine, pinitol, muco-inositol, chiro-inositol, and chlorogenic acid; ornithine, muco-inositol, chiro-inositol, betaine, and chlorogenic acid; ornithine, pinitol, chiro-inositol, betaine, and chlorogenic acid; ornithine, pinitol, muco-inositol, betaine, and chlorogenic acid; or pinitol, muco-inositol, chiro-inositol, betaine, and chlorogenic acid. The composition may include at least one of ornithine, pinitol, muco-inositol, chiro-inositol, betaine, and chlorogenic acid. The six compounds in the composition may be in a weight ratio of about 1 to about 5: about 4 to about 35: about 1 to about 8: about 2 to about 16: about 2 to about 20: about 2 to about 16. When the composition includes at least one of ornithine, pinitol, muco-inositol, chiro-inositol, betaine, and chlorogenic acid, each of these compounds be present in an amount of about 0.1 wt% to about 4 wt%, about 0.6 wt% to about 25 wt%, about 0.2 wt% to about 8 wt%, about 0.3 wt% to about 16 wt%, about 0.3 wt% to about 24 wt%, or about 0.2 wt% to about 12 wt%, with respect to a total weight of the composition.

In the composition for use the at least four compounds selected from ornithine, pinitol, muco-inositol, chiro-inositol, betaine, and chlorogenic acid may be additionally combined with at least one compound selected from linoleic acid, palmitic acid, and α-linolenic acid. Accordingly, the at least four compounds selected from ornithine, pinitol, muco-inositol, chiro-inositol, betaine, and chlorogenic acid may additionally be combined with linolenic acid; palmitic acid; α-linolenic acid; linoleic acid and palmitic acid; linoleic acid and α-linolenic acid; palmitic acid and α-linolenic acid; or linoleic acid, palmitic acid, and α-linolenic acid. The composition for use may include α-linolenic acid, linoleic acid, and palmitic acid in a weight ratio of about 1 to about 30: about 1 to about 15: about 1 to about 15. The composition for use may include α-linolenic acid, linoleic acid, and palmitic acid in an amount of about wt% to about 30 wt%, about 1 wt% to about 15 wt%, and about 1 wt% to about 15 wt%, respectively, with respect to a total weight of the composition.

The composition for use may be a pharmaceutical composition including the *Artemisia annua* extract. The composition for use may be a drug or medical formulation in which is included a pharmaceutically acceptable carrier for the *Artemisia annua* extract to be administered into a subject. The term 'pharmaceutical' means having pharmacological activity and includes the use of the composition according to one or more embodiments as a pharmaceutical composition for use in the treatment of diseases according to the claims. Accordingly, the composition for use may be in the form of functional foods, cosmetics, quasi-drugs, or external skin preparations for skin health.

The 'pharmaceutically acceptable salt' may mean an organic or inorganic salt of a compound in the composition for use which is comparatively non-toxic to patients and at a harmless effective concentration, and side effects caused by the salt do not impair the beneficial efficacy of compounds in the composition. These salts may use an inorganic acid or an organic acid as a free acid. Hydrochloric acid, bromic acid, nitric acid, sulfuric acid, perchloric acid, phosphoric acid, or the like may be used as the inorganic salt. Citric acid, acetic acid, lactic acid, maleic acid, fumaric acid, gluconic acid, methanesulfonic acid, glyconic acid, succinic acid, tartaric acid, galacturonic acid, embonic acid, glutamic acid, aspartic acid, oxalic acid, (D) or (L) malic acid, maleic acid, methanesulfonic acid, ethanesulfonic acid, 4-toluenesulfonic acid, salicylic acid, citric acid, benzoic acid, or malonic acid may be used as the organic acid. These salts may include an alkali metal salt (sodium salt, potassium salt, and the like) and an alkali earth metal salt (calcium salt, magnesium salt, and the like. For example, acid salts may include acetate, aspartate, benzoate, besylate, bicarbonate/carbonate, bisulfate/sulfate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulfate, naphthylate, 2-napsylate, nicotinate, nitrate, orotate, oxalate, palmitate, pamoate, phosphate/hydrogen phosphate/dihydrogen phosphate, saccharate, stearate, succinate, tartrate, tosylate, trifluoroacetate, aluminum, arginine, benzathine, calcium, choline, diethylamine, diolamine, glycine, lysine, magnesium, meglumine, olamine, potassium, sodium, tromethamine, a zinc salt, or the like. For example, hydrochloride or trifluoroacetate of these salts may be used as an acid salt.

The subject may be a mammal, for example, a human, a cow, a horse, a pig, a dog, a sheep, a goat, or a cat. For example, the mammal may be a human. An effective dosage of the composition for use for a human body may vary depending on the age, weight sex, health conditions, and disease severity of a patient, and a dosage form.

The administration may be oral administration or parenteral administration such as intravenous, intraperitoneal, intracutaneous, subcutaneous, epithelial, or muscular administration. Through these administration routes, the composition according to any of the embodiments may be administered in a form of various medical formulations. When the composition for use is prepared as a formulation, a diluent or an excipient, for example, a filler, an extender, a binder, a wetting agent, a disintegrant, a surfactant, or the like may be used.

Solid formulations for oral administration may include tablets, pills, powders, granules, capsules, troches, and the like. These solid formulations may be prepared by mixing at least one compound with at least one excipient, for example, starch, calcium carbonate, sucrose, lactose, or gelatin. In addition to simple excipients, lubricants such as magnesium stearate talc may be used. Liquid formulations for oral administrations may include suspensions, enteri liquid, emulsions, or syrups. Liquid formulations may include, in addition to commonly used simple diluents such as water or liquid paraffin, a variety of excipients, for example, a wetting agent, a sweetener, a flavoring agent, a preservative, or the like.

Formulations for parenteral administration may include a sterile aqueous solution, a non-aqueous solvent, a suspending agent, an emulsion, a lyophilized formulation, a suppository, or the like. Non-aqueous solvents or suspending agents may include propylene glycol, polyethylene glycol, vegetable oils such as olive oil, or injectable esters such as ethyl oleate. Bases for suppositories may be witepsol, macrogol, Tween 61, cacao butter, laurin butter, glycerol, gelatin, or the like.

In particular, dermatologic external preparations for application to the skin may be applicable in the form of a patch, a band, an emulsion, an ointment, a pack, a gel, a cream, a lotion, a liquid, or powder. Cosmetics may be applicable in the form of a softening liquid, a nutritional liquid, a massage cream, a nutritional cream, a moisturizing cream, a functional lotion, a mist, a pack, a gel, or a skin adhesive type formulation. Accordingly, for use as such dermatologic external preparations, the composition for use may be mixed with common ingredients used in dermatologic external preparations such as cosmetics or medical supplies, for example, an aqueous ingredient, an oily ingredient, a powder ingredient, alcohols, a moisturizer, a thickening agent, an ultraviolet absorbent, a whitening agent, an antiseptic, an antioxidant, a surfactant, a fragrance ingredient, a coloring agent, or various skin nutrients, in an appropriate ratio according to a need.

In the dermatologic external preparations, a metal chelating agent such as disodium edentate, trisodium edentate, sodium citrate, sodium polyphosphate, sodium metaphosphate, gluconic acid, or the like; caffeine, tannin, verapamil, glabridin licorice extract, hot-water extract of carin fruits, various herb medicines, tocopherol acetate, glycyrrhetinic acid, tranexamic acid, and derivatives or salts thereof; vitamin C, magnesium ascorbyl phosphate, ascorbyl glucoside, arbutin, kojic acid; and saccharides such as glucose, fructose, and trehalose may be appropriately mixed.

According to another aspect of the disclosure, there is provided a medicine for use in the prevention or treatment of a skin disorder in an animal, the medicine including the *Artemisia annua* extract prepared using the method of isolating the *Artemisia annua* extract as an active ingredient, wherein the skin disorder is at least one inflammatory skin disorder selected from the group consisting of skin pruritus, seborrheic dermatitis, contact dermatitis, deciduous dermatitis, hidradenitis, atopic dermatitis, drug allergy, diabetic dermatitis, bacterial dermatitis, fungal dermatitis, allergic dermatitis and neurogenic dermatitis.

The animal may be a mammal, a bird, fish, or a reptile. The animal may be livestock, a pet, or aquaculture fish. For example, the animal may be at least one kind of animal selected from the group consisting of chickens, pigs, cows, horses, dogs, rabbits, cats, ducks, goats, and sheep.

The medicine for animals may be used as a sole medicine, or may be prepared and used as an additive for animal feed. The medicine for animals as an additive for animal feeds may be in the form of powder, granules, pellets, liquid, or suspension. The medicine for animals may be supplied alone or by being mixed with animal feed.

According to another aspect of the disclosure, there is provided a food composition for use in the prevention or amelioration of a skin disorder, the food composition including the *Artemisia annua* extract prepared by the method of isolating an *Artemisia annua* extract as an active ingredient. The food composition may further include an excipient or carrier in food acceptable level. The food composition may be a health functional food.

The health functional food as defined herein may be a health functional food as newly defined in the Act on Health Functional Foods revised in 2008, whose functionality and safety in the human body have been sufficiently established and is included in the regulations on functional raw material qualification for health functional foods prescribed in Korean Food and Drug Administration Notice No. 2008-72.

When the composition for use is a health functional food, the composition may be added as it is, or may be used along with other health functional foods or health functional food ingredients, or may be appropriately used according to a usual method. A mixed amount of an active ingredient(s) may be determined to be appropriate for the purpose of use. In general, the amount of the active ingredient included in the composition according to one or more embodiments may be about 0.01 wt% to about 15 wt%, for example, about 0.2 wt% to about 10 wt% with respect to a total weight of the food. When a beverage is prepared, based on 100 mL of the beverage, about 0.1 g to about 30 g, for example, about 0.2 g to about 5 g of the active ingredient in the composition may be contained. The beverage may entirely consist of natural ingredients. However, in the case of long-term intake for health control and hygienic purposes, the amount of the active ingredient may be equal to or below the above ranges. The active ingredient may be used in an amount equal to or above the ranges, since there are no problems in terms of safety.

The composition for use in the form of health functional foods may be formulated as a usual form of health functional food known in the art. The health functional food may be prepared as, for example, powder, granules, tablets, pills, capsules, suspensions, emulsions, syrups, infusions, liquids, extract, gums, teas, jellies, or beverage. For example, the composition may be formulated as a beverage. The carrier or additive in food acceptable level may be any carrier or additive known in the art to be available for a formulation to be prepared. The health functional food may include a food available as animal feed.

The health functional food may contain nutrients, vitamins, electrolytes, flavoring agents, coloring agents, pectic acid and salts thereof, alginic acid and salts thereof, organic acids, protective colloid thickening agents, pH adjusting agents, stabilizers, preservatives, glycerin, alcohols, carbonating agents used in carbonated beverages, or the like according to purposes or preferences. In preparation of other natural fruit juices, fruit juice beverages, and vegetable beverages, flesh may be contained. The composition for health functional foods may further include a food additive. Unless prescribed otherwise, acceptability as a "food additive" may be determined based on the specification of an item of concern and standards therefor according to the general provisions and general test methods prescribed in the Korean Food Additives Codex approved by the Korea Ministry of Food and Drug Safety.

According to another aspect of the disclosure, there is provided a quasi-drug composition for use in the prevention or amelioration of an inflammatory skin disorder, the composition including the *Artemisia annua* extract prepared according to the above-described method of isolating an *Artemisia annua* extract as an active ingredient. The quasi-drug composition may further include an excipient or carrier in quasi-drug acceptable level.

The term 'quasi-drug' means a pharmaceutical product that does not include products for use as medicines as defined by the Pharmaceutical Affairs Act, but rather defined separately by the classification criteria set by the Korea Ministry of Health and Welfare for products with a minor effect on human bodies in comparison with medicines for use in treatment or prevention of diseases. Accordingly, quasi-drugs may include fiber/rubber products for use in the treatment or prevention of diseases in humans or animals, products which are not or similar to instrument or machine and have a minor effect or no direct action on the human body, or sterilizers/insecticides for prevention of infectious diseases. In one or more embodiments, the quasi-drug may be a product for moisturizing, preventing or treating skin pruritus or atopic dermatitis.

According to another aspect of the disclosure, there is provided a cosmetic composition for use in the prevention or amelioration of an inflammatory skin disorder, the cosmetic composition including the *Artemisia annua* extract prepared according to the above-described method of isolating an *Artemisia annua* extract as an active ingredient. The cosmetic composition for use may further include a cosmetically acceptable excipient or carrier. The cosmetic composition for use may be a functional cosmetic.

According to the revision of the Enforcement Regulations of the Cosmetic Act, legislation of which was pre-announced by the Korea Ministry of Food and Drug Safety on August 11, 2016, an atopic skin moisturizing function is added to functional cosmetics. In one or more embodiments, the functional cosmetic is for use in the prevention or treatment of atopic dermatitis.

## Claims

1. A method of isolating an *Artemisia annua* extract, the method comprising:
contacting *Artemisia annua* plant with a low-polarity solvent to extract a component of the *Artemisia annua* into the low-polarity solvent, wherein the low polarity solvent is dichloromethane, diethyl ether, ethyl acetate or acetone;
removing the low-polarity solvent from a mixture obtained after the contacting to obtain a residue; and
contacting the residue with a hydrophilic solvent to extract a component of the *Artemisia annua* into the hydrophilic solvent, wherein the hydrophilic solvent is a C1-C6 alcohol, water, or a combination thereof, and
wherein the *Artemisia annua* extract comprises at least four compounds selected from the group consisting of ornithine, pinitol, muco-inositol, chiro-inositol, betaine, and chlorogenic acid.

2. The method of claim 1, further comprising contacting the *Artemisia annua* extract with a fractionating solvent to fractionate the *Artemisia annua* extract, wherein the fractionating solvent is water, a C1-C6 alcohol or a combination thereof.

3. The method of claim 2, wherein the fractionating comprises contacting the *Artemisia annua* extract with a chromatography medium to be bound thereto and eluting the *Artemisia annua* extract with a fractionating solvent.

4. The method of claim 2, wherein the fractionation comprises elution with a fractionating solvent containing 20 vol% or greater to less than 40 vol%, 40 vol% or greater to less than 60 vol%, 60 vol% or greater to less than 100 vol%, or about 100 vol% of alcohol with respect to a total volume of the fractionating solvent.

5. A composition for use in the prevention or treatment of a skin disorder, the composition comprising the *Artemisia annua* extract prepared by the method of claim 1,
wherein the skin disorder is at least one inflammatory skin disorder selected from the group consisting of skin pruritus, seborrheic dermatitis, contact dermatitis, deciduous dermatitis, hidradenitis, atopic dermatitis, drug allergy, diabetic dermatitis, bacterial dermatitis, fungal dermatitis, allergic dermatitis and neurogenic dermatitis, and
wherein the *Artemisia annua* extract comprises at least four compounds selected from the group consisting of ornithine, pinitol, muco-inositol, chiro-inositol, betaine, and chlorogenic acid.

6. The composition for use according to claim 5, wherein the *Artemisia annua* extract comprises 0.01 % (w/w) or less of sesquiterpene lactone or does not comprise sesquiterpene lactone.

7. The composition for use according to claim 5, wherein the *Artemisia annua* extract further comprises at least one compound selected from the group consisting of linoleic acid, palmitic acid, and α-linolenic acid.

8. The composition for use according to claim 5, wherein the *Artemisia annua* extract is a combination of a fraction obtained by fractionation with a solvent including 20 vol% or greater to less than 40 vol% of alcohol with respect to a total volume of the solvent; and a fraction obtained by fractionation with a solvent of 100 vol% of alcohol.

9. The composition for use according to claim 5, wherein the composition is a pharmaceutical composition, a quasi-drug composition, a food composition, or a cosmetic composition.

## Patentansprüche

1. Verfahren zum Isolierung eines Extrakts von *Artemisia annua,* das Verfahren umfassend: Inkontaktbringen der Pflanze *Artemisia annua* mit einem niedrigpolaren Lösungsmittel, um einen Bestandteil der *Artemisia annua* in das niedrigpolare Lösungsmittel zu extrahieren, wobei das niedrigpolare Lösungsmittel Dichlormethan, Diethylether, Ethylacetat oder Aceton ist;
Entfernen des niedrigpolaren Lösungsmittels aus einem Gemisch, das nach dem Inkontaktbringen erlangt wird, um einen Rückstand zu erlangen; und
Inkontaktbringen des Rückstands mit einem hydrophilen Lösungsmittel, um einen Bestandteil der *Artemisia annua* in das hydrophile Lösungsmittel zu extrahieren, wobei das hydrophile Lösungsmittel ein C1-C6-Alkohol, Wasser oder eine Kombination davon ist, und
wobei der Extrakt von *Artemisia annua* mindestens vier Verbindungen umfasst, die ausgewählt sind aus der Gruppe, bestehend aus Ornithin, Pinitol, Muco-Inositol, Chiro-Inositol, Betain und Chlorogensäure.

2. Verfahren nach Anspruch 1, ferner umfassend ein Inkontaktbringen des Extrakts von *Artemisia annua* mit einem fraktionierenden Lösungsmittel, um den Extrakt von *Artemisia annua* zu fraktionieren, wobei das fraktionierende Lösungsmittel Wasser, ein C1-C6-Alkohol oder eine Kombination davon ist.

3. Verfahren nach Anspruch 2, wobei das Fraktionieren ein Inkontaktbringen des Extrakts von *Artemisia annua* mit einem daran zu bindenden Chromatographiemedium und ein Eluieren des Extrakts von *Artemisia annua* mit einem fraktionierenden Lösungsmittel umfasst.

4. Verfahren nach Anspruch 2, wobei die Fraktionierung eine Elution mit einem fraktionierenden Lösungsmittel umfasst, das 20 Volumenprozent oder mehr bis weniger als 40 Volumenprozent, 40 Volumenprozent oder mehr bis weniger als 60 Volumenprozent, 60 Volumenprozent oder mehr bis weniger als 100 Volumenprozent oder etwa 100 Volumenprozent Alkohol, bezogen auf ein Gesamtvolumen des fraktionierenden Lösungsmittels, enthält.

5. Zusammensetzung zur Verwendung bei der Vorbeugung oder Behandlung einer Hauterkrankung, wobei die Zusammensetzung den nach dem Verfahren von Anspruch 1 hergestellten Extrakt von *Artemisia annua* umfasst,
wobei die Hauterkrankung mindestens eine entzündliche Hauterkrankung ist, die ausgewählt ist aus der Gruppe, bestehend aus Hautpruritus, seborrhoischer Dermatitis, Kontaktdermatitis, Laubdermatitis, Hidradenitis, atopischer Dermatitis, Arzneimittelallergie, diabetischer Dermatitis, bakterieller Dermatitis, Pilzdermatitis, allergischer Dermatitis und neurogener Dermatitis, und
wobei der Extrakt von *Artemisia annua* mindestens vier Verbindungen umfasst, die ausgewählt sind aus der Gruppe, bestehend aus Ornithin, Pinitol, Muco-Inositol, Chiro-Inositol, Betain und Chlorogensäure.

6. Zusammensetzung zur Verwendung nach Anspruch 5, wobei der Extrakt von *Artemisia annua* 0,01 % (nach Gewicht) oder weniger Sesquiterpenlacton umfasst oder kein Sesquiterpenlacton umfasst.

7. Zusammensetzung zur Verwendung gemäß Anspruch 5, wobei der Extrakt von *Artemisia annua* ferner mindestens eine Verbindung umfasst, die ausgewählt ist aus der Gruppe, bestehend aus Linolsäure, Palmitinsäure und α-Linolensäure.

8. Zusammensetzung zur Verwendung nach Anspruch 5, wobei der Extrakt von *Artemisia annua* eine Kombination einer Fraktion, die durch Fraktionieren mit einem Lösungsmittel erlangt wird, das 20 Volumenprozent oder mehr bis weniger als 40 Volumenprozent Alkohol, bezogen auf das Gesamtvolumen des Lösungsmittels, beinhaltet, und einer Fraktion, die durch Fraktionieren mit einem Lösungsmittel mit 100 Volumenprozent Alkohol erlangt wird, ist.

9. Zusammensetzung zur Verwendung gemäß Anspruch 5, wobei die Zusammensetzung eine pharmazeutische Zusammensetzung, eine arzneimittelähnliche Zusammensetzung, eine Nahrungsmittelzusammensetzung oder eine kosmetische Zusammensetzung ist.

## Revendications

1. Procédé d'isolement d'un extrait d'*Artemisia annua,* le procédé comprenant : la mise en contact de la plante *Artemisia annua* avec un solvant à faible polarité pour extraire un composant d'*Artemisia annua* dans le solvant à faible polarité, le solvant à faible polarité étant le dichlorométhane, l'éther diéthylique, l'acétate d'éthyle ou l'acétone ;
l'élimination du solvant à faible polarité d'un mélange obtenu après la mise en contact pour obtenir un résidu ; et
la mise en contact du résidu avec un solvant hydrophile pour extraire un composant d'*Artemisia annua* dans le solvant hydrophile, le solvant hydrophile étant un alcool en C1-C6, l'eau ou une combinaison de ceux-ci, et
l'extrait d'*Artemisia annua* comprenant au moins quatre composés choisis parmi le groupe constitué d'ornithine, pinitol, muco-inositol, chiro-inositol, bétaïne et acide chlorogénique.

2. Procédé de la revendication 1, comprenant en outre la mise en contact de l'extrait d'*Artemisia annua* avec un solvant de fractionnement pour fractionner l'extrait d'*Artemisia annua,* dans lequel le solvant de fractionnement est l'eau, un alcool en C1-C6 ou une combinaison de ceux-ci.

3. Procédé de la revendication 2, dans lequel le fractionnement comprend la mise en contact de l'extrait d'*Artemisia annua* avec un milieu de chromatographie pour y être lié et l'élution de l'extrait d'*Artemisia annua* avec un solvant de fractionnement.

4. Procédé de la revendication 2, dans lequel le fractionnement comprend l'élution avec un solvant de fractionnement contenant 20 % en volume ou davantage à moins de 40 % en volume, 40 % en volume ou davantage à moins de 60 % en volume, 60 % en volume ou davantage à moins de 100 % en volume, ou environ 100 % en volume d'alcool par rapport au volume total du solvant de fractionnement.

5. Composition destinée à être utilisée dans la prévention ou le traitement d'une affection cutanée, la composition comprenant l'extrait d'*Artemisia annua* préparé selon le procédé de la revendication 1,
l'affection cutanée étant au moins une affection cutanée inflammatoire choisie parmi le groupe constitué de prurit cutané, dermatite séborrhéique, dermatite de contact, dermatite exfoliative, hidradénite, dermatite atopique, allergie médicamenteuse, dermatite diabétique, dermatite bactérienne, dermatite fongique, dermatite allergique et dermatite neurogène, et
dans laquelle l'extrait d'*Artemisia annua* comprend au moins quatre composés choisis parmi le groupe constitué d'ornithine, pinitol, muco-inositol, chiro-inositol, bétaïne et acide chlorogénique.

6. Composition destinée à être utilisée selon la revendication 5, dans laquelle l'extrait d'*Artemisia annua* comprend 0,01 % (p/p) ou moins de sesquiterpène lactone ou ne comprend pas de sesquiterpène lactone.

7. Composition destinée à être utilisée selon la revendication 5, dans laquelle l'extrait d'*Artemisia annua* comprend en outre au moins un composé choisi parmi le groupe constitué d'acide linoléique, acide palmitique et acide α-linolénique.

8. Composition destinée à être utilisée selon la revendication 5, dans laquelle l'extrait d'*Artemisia annua* est une combinaison d'une fraction obtenue par fractionnement avec un solvant comprenant 20 % en volume ou davantage à moins de 40 % en volume d'alcool par rapport au volume total du solvant ; et une fraction obtenue par fractionnement avec un solvant à 100 % en volume d'alcool.

9. Composition destinée à être utilisée selon la revendication 5, la composition étant une composition pharmaceutique, une composition quasi-médicamenteuse, une composition alimentaire ou une composition cosmétique.
